# EUROPÄISCHE PATENTSCHRIFT

(11) **EP 2 125 086 B1**
(45) Veröffentlichungstag und Bekanntmachung des Hinweises auf die Patenterteilung: **07.11.2012**
(21) Anmeldenummer: 08700277.0
(22) Anmeldetag: 16.01.2008
(51) Int. Cl.: A61M 5/46, A61M 5/32, A61M 5/42, A61M 5/50, A61M 5/24

(54) **EINWEG-INJEKTIONSGERÄT**
DISPOSABLE INJECTION APPARATUS
APPAREIL D'INJECTION À USAGE UNIQUE

(30) Priorität: 17.01.2007 AT 862007
(43) Veröffentlichungstag der Anmeldung: 02.12.2009
(73) Patentinhaber: Jütte, Heidi Annemarie, 1060 Wien (AT); Jütte, Astrid, 1090 Wien (AT); Jütte, Wilhelm, 1150 Wien (AT); Jütte, Margrit, 1060 Wien (AT)
(72) Erfinder: Die Erfindernennung liegt noch nicht vor
(86) Internationale Anmeldenummer: PCT/AT2008/000010
(87) Internationale Veröffentlichungsnummer: WO 2008/086552

(56) Entgegenhaltungen:
- EP-A- 1 410 818
- AT-B- 411 222
- DE-A1- 19 604 838
- DE-C1- 19 900 792
- FR-A- 2 762 790
- US-A- 159 192
- US-A- 2 531 267
- US-A- 5 609 577
- US-B1- 6 830 560

## Beschreibung

Die Erfindung betrifft ein Einweg- Injektionsgerät mit einem Behälter für Injektionsmedien, welcher von einem Ende einer an beiden Enden zugespitzten Kanüle durchstechbar ist, und mit einer Einrichtung zur irreversiblen Fixierung der Kanüle in einem Gehäuse nach Gebrauch des Injektionsgerätes.

Ein solches Gerät eignet sich insbesondere zur Durchführung von subkutanen, aber beispielsweise auch für intramuskuläre Injektionen.

In der Human- und Veterinärmedizin sind trotz anderer Neuentwicklungen subkutane Injektionen noch immer der beste Weg, eine bestimmte Dosis eines Arzneimittels rasch und präzise in einer definierten Region des Unterhautzellgewebes oder eines Muskels zu deponieren. Nach derzeitigem Stand ist es besonders wünschenswert, dass diese Injektionen - speziell bei der Selbstbehandlung chronisch Kranker - einfach, sicher, ohne psychischen Stress, möglichst schmerzfrei, ohne Entstehen von gefährlichem Abfall und womöglich mit einer in Stufen einstellbaren Dosis und/oder Einstichtiefe durchgeführt werden können. Ebenso ist anzustreben, dass auch sehschwache Patienten, die auf regelmäßige Wirkstoffgaben angewiesen sind, diese Injektionen selbst durchführen können. Es ist daher notwendig, Einweg-Injektionsgeräte zu schaffen, die möglichst alle der genannten Anforderungen erfüllen.

Weiterentwicklungen sind vor allem bei vorgefüllten Einwegspritzen oder mehrfach verwendbaren Spritzampullen, so genannten "Pens", wünschenswert Bei neueren Lösungen sind bereits einige, den praktischen Gebrauch betreffende Fortschritte erzielt worden (J. Blair Polin: "The Ins and Outs of Prefilled Syringes" in: Pharmaceutical Medical Packaging News, Mai 2003). Es mangelt aber weiterhin an einem technischen Konzept, mit welchem in einer einzigen Injektionsvorrichtung, die zugleich als vorgefüllte Einwegspritze und als Sicherheitsspritze ausgelegt ist, bei der Selbst oder Fremdverabreichung von subkutanen oder intramuskulären Infektionen möglichst viele der heute für eine medikationsgerechte Applikation zusätzlich notwendigen Anforderungen realisiert werden können (Ian Thompson: "New-generation auto-Injectors" in: "Prefilled syringes: innovations that meet the growing demand", ONdrugDelivery Ltd. 2005).

Einige Verbesserungsaspekte werden von der in AT 411 222 B beschriebenen Einweg-Injektionsvorrichtung verwirklicht. In einer Nadelkammer mit flexibler Seitenwand befindet sich unterhalb einer aus plastisch bis elastisch deformierbarem Material gefertigten Vorratskammer mit dem Injektionsmedium eine Nadelhalterung mit doppelt zugeschärfter Injektionsnadel, deren oberes Ende nach Erreichen der Stichtiefe im Gewebe in die Vorratskammer eindringt, worauf das Injektionsmedium in das Gewebe befördert wird. Dies erfolgt durch Druckausübung auf die Vorratskammer. Zugleich wird ein Federbügel von einer Nadelbühne im Inneren der Nadelkammer geschoben, welcher nach dem Zurückziehen des unteren Nadelendes in die Nadelkammer deren Nadelöffnung irreversibel verschließt An der Unterseite der Vorrichtung kann sich eine Adhäsivschicht zur Fixierung der Vorrichtung auf der Hautoberfläche befinden, um eine möglichst verletzungsfreie Bewegung der Nadel im Gewebe zu erreichen.

Die bekannte Injektionsvorrichtung zielt somit bereits darauf ab, Verletzungen mit der Injektionsnadel durch unachtsames Hantieren, Läsionen des Bindegewebes durch Zerschneiden desselben bei freihändigem Einstechen und psychischen Stress aus Angst vor Selbstverletzung (Nadelphobie, insbesondere von Kindern) zu vermeiden. Es kann vor allem auch kein gefährlicher Abfall mehr entstehen (Möglichkeit der Sekundärinfektion durch bereits benützte, infizierte Nadeln).

Darüber hinaus wären allerdings weitere Annehmlichkeiten von großem Vorteil, die in den heute bekannten Injektionsvorrichtungen weder insgesamt noch zusätzlich zu den voran stehend bereits erwähnten Verbesserungen anzutreffen sind. Hierzu zählt beispielsweise die einfache und sichere visuelle Überprüfbarkeit bestimmter Injektionslösungen, z.. B. von Heparinlösungen, auf Verfärbungen und/oder Trübungen vor Gebrauch. Diese Überprüfung ist bei sichthemmender Abschirmung des Medikamenten- Behälters durch Halterungen oder Kappen und/oder wegen eines lichtstreuenden Wandmaterials des Behälters bzw. der Vorratskammer - z.. B. Polyethylen oder Polypropylen - nicht immer einwandfrei möglich. Eine weitere Anforderung ist die Milderung einer nicht von der Beschaffenheit der Injektionsnadel abhängigen Schmerzempfindung, wenn also die Nadel z. B. auf einen Nerv trifft

Für manche Injektionsmedien wird die Möglichkeit einer - beispielsweise vom Körpergewicht abhängigen - sicheren und exakten Abstufung der abgegebenen Dosis gefordert, was bei den bekannten Ausführungsformen nicht möglich ist. In manchen Fällen - beispielsweise wenn die Körperoberfläche nass und/oder stark behaart ist oder die injektion in Notfallsituationen durch leichte Bekleidung hindurch erfolgen soll - ist auch die Fixierung der Vorrichtung über der Injektionsstelle mittels eines Adhäsivs allein nicht ausreichend sicher, da die Vorrichtung verrutschen kann.

Not- und Katastrophenfälle erfordern die Bereitstellung von Injektionsvorrichtungen, die eine einfache und sichere subkutane bzw. intramuskuläre Selbst- oder Fremdverabreichung von überlebenswichtigen Injectabilia auch ohne Assistenz von speziell ausgebildetem medizinischem Fachpersonal ermöglichen.

Unzureichend gelöst ist ferner das Problem der sicheren Selbstbehandlung mit herkömmlichen Injektionsgeräten - insbesondere von Patienten mit Sehschwäche, geringer manueller Geschicklichkeit versteiften Handgelenken oder Tremor - bei gleichzeitiger Kontrolle variabler Einstellungen durch taktil wahrnehmbare Signale.

Von Vorteil wäre es schließlich, auch die maximale Eindringtiefe der injektionsnadel in das Gewebe den individuellen Gegebenheiten anpassen zu können, d. h. die Stichtiefe bei einer solchen Vorrichtung einfach zu variieren und sicher zu begrenzen.

Darüber hinaus sollte in Abhängigkeit von der Medikation und auch von der Empfindung des Patienten - selbst nach längerer Lagerung des vorgefüliten Behälters oder bei höherviskosen Injektionsflüssigkeiten - beim Injizieren ein ruckfreier Beginn des Injektionsvorgangs ermöglicht werden und die Dosiergeschwindigkeit bzw. die Dauer des Injektionsvorgangs einfach und sicher variiert werden können.

Generell wird für vorgefülite Injektionsvorrichtungen, die besonders empfindliche Injektionsmedien enthalten, eine verbesserte Lagerungsfähigkeit, welche unter anderem auch von der Beschaffenheit der Injektionsvorrichtung selbst abhängt, gewünscht. Die Werkstoffe der Behälterwand bestimmen beispielsweise deren Verträglichkeit und eventuelle Wechselwirkung mit den Inhaltsstoffen der Injektionslösung. Bei Behältern aus Kunststoff mit flexibler, ein- oder mehrschichtig gefertigter Wand kann die materialbedingte Durchlässigkeit für Wasserdampf und/oder Sauerstoff manchmal die Lagerfähigkeit entscheidend begrenzen. Schließlich ist auch die Unempfindlichkeit des Dosiermechanismus und des Sicherheitsmechanismus gegenüber Schlag, Stoß oder Fall - beispielsweise beim Transport verpackter Injektionsvorrichtungen oder bei ungeschickter Handhabung - eine weitere, praktisch wichtige Forderung.

Um nun möglichst alle genannten Verbesserungen in einem Injektionsgerät zu implementieren, bedarf es eines neuartigen technischen Konzepts. Ziel der vorliegenden Erfindung ist es daher, die erwähnten Mängel bekannter Injektionsvorrichtungen zu überwinden und durch spezielle Maßnahmen eine Vielzahl von Vorteilen im Vergleich zu derzeit üblichen Spritzampullen zu erzielen.

Das erfindungsgemäße Einweg- Injektionsgerät ist dadurch gekennzeichnet, dass
- der Behälter in einer Aufnahme gehaltert ist, welche in dem Gehäuse drehbar und in Richtung der Kanülenachse verschiebbar ist,
- in der Seitenwand des Gehäuses zumindest ein gewindegangförmiger Spalt zur Führung eines mit dem Sockel der Aufnahme verbundenen Fingerhebels vorgesehen ist, wobei der Sockel entweder ein Steuerelement oder eine Komplementäreinheit für das Steuerelement trägt,
- im Inneren des Gehäuses koaxial und unterhalb der Aufnahme eine in Richtung der Kanülenlachse verschiebbare, aber nicht um die Kanülenachse drehbare Kanülenhalterung angeordnet ist, welche Kanülenhalterung entweder eine mit dem Steuerelement auf dem Sockel zusammenwirkende Komplementäreinheit oder ein zu der Komplementäreinheit auf dem Sockel korrespondierendes Steuerelement aufweist,
- am unteren Ende der Kanülenhalterung eine Druckscheibe ausgebildet ist, die unter dem ständigen Druck eines zwischen dem Boden des Gehäuses und der Druckscheibe angeordneten elastischen Elementes steht, dass
- der untere Bereich des Gehäuses mit einer Bodenplatte verbunden ist, wobei Gehäuseboden und Bodenplatte jeweils eine zentrale Kanülenöffnung aufweisen, und dass um die Seitenwand des Gehäuses ein offener Stellring mit wenigstens einem von außen durch zumindest ein Fenster in der Seitenwand eingreifenden keilförmigen Riegel vorgesehen ist, welcher Riegel die Druckscheibe und damit die Kanülenhalterung vor Gebrauch des Gerätes in einer Ausgangsposition hält.

Der Stellring kann außen geriefelt sein, um ein Abrutschen der Finger zu vermeiden. Er weist meist zwei elastische Zungen mit jeweils einem, zunächst am Rand der Druckscheibe der Kanülenhalterung aufliegenden, in zwei Richtungen keilförmig abgeschrägten Riegel auf.

Die Riegel können als Doppelriegel ausgeführt sein, um bei bestimmten Ausführungsformen des Injektionsgerätes auch eine Abwärtsbewegung der Kanüle gegen den Druck des elastischen Elementes vor der durch Verdrehen des Stellringes willentlich herbeigeführten Freigabe der Kanülenhalterung sicher zu verhindern. In diesem Fall wird der Rand der Druckscheibe von oben und von unten fixiert. Der obere Teil eines solchen Doppelriegels ist gleichfalls in zwei Richtungen keilförmig abgeschrägt, der untere Teil stellt einen einfachen Keil dar.

Der Stellring weist vorteilhaft zusätzlich eine Hebelsperre auf. Die im wesentlichen trapez- oder dreieckförmige Hebelsperre kann je nach Ausführung des Injektionsgerätes vom Stellring nach oben ragen ("hochgestellt") oder als eine vom oberen Rand des Stellringes ausgehende Ausnehmung ("tiefgestellt") gestaltet sein. Diese Sperreinrichtung dient zur Begrenzung bzw. Blockierung der Abwärtsbewegung des Fingerhebels an bestimmten Stellen seiner Führung; auf diese Weise können genau definierte Teilmengen an Injektionsmedien aus dem Behälter abgegeben werden. Zu diesem Zweck trägt der Stellring auch eine mit auf dem Gehäuse vorgesehenen Stellmarken zur Dosiseinstellung zusammenwirkende Markierung. Sind an der Außenseite des Gehäuses Stellmarken zur Einstellung der aus dem Behälter abgegebenen Dosis angebracht, so können die Riegel gleichzeitig in entsprechende Blindfenster auf der Behälteraußenwand einrasten, wenn die - beispielsweise pfeilförmige - Markierung des mit einer Hebelsperre versehenen Stellringes auf bestimmte Stellmarken zeigt.

Als Behälter für das Injektionsmedium können zweckmäßig zylindrische, einseitig offene Gefäße eingesetzt werden, wie beispielsweise vorgefüllte Karpulen, deren verschiebbarer, flexibler Kolbenstopfen zur oberen Kanülenspitze gerichtet ist Die Karpulen (z. B. mit einem Volumen von 0,5 mL Injektionsmedium) sind beispielsweise aus Spritzenglas oder aus einem glasklaren Cycloalken-Copolymerisat gefertigt. Die Karpulen weisen vorteilhaft handelsübliche Dimensionen auf, die Verschluss-Systeme (z. B. Kolbenstopfen) bestehen aus handelsüblichen, auf das Injektionsmedium abgestimmten Elastomeren oder Verbundwerkstoffen. Ein Kolbenstopfen mit zentraler Bohrung, in welcher sich darüber hinaus ein hülsenförmiger Einsatz zur Versteifung befinden kann, gelangt derzeit bei handelsüblichen Karpulen nicht zum Einsatz.

Kunststoffbehälter mit deformierbarer Wand - beispielsweise in Form einer Kugel, eines Drehellipsoids oder eines zylindrischen Balgs - mit Kapselvorsatz aus Glas oder glasklarem Kunststoff sind gleichfälls als Behälter für Injektionsmedien geeignet. Ein solcher Behälter wird beispielsweise nach dem BFS ( blow-fill-seal) - Verfahren hergestellt und zugleich steril abgefüllt. Beispielsweise wird eine Kunststoffkapsel in Form eines Drehellipsoids einschließlich Kapselvorsatz, ähnlich wie eine Karpule, zweckmäßig von oben in die Aufnahme eingesetzt und dort von einer unteren Klammer der Behälterfassung - einem Teil der Aufnahme - festgehalten oder von unten in einen Klemmsitz der Aufnahme gedrückt. Nach dem Durchstechen der Kapselwand an der Unterseite der Kapsel wird dieser Teil der Kapselwand von einer Kanülenfassung zunehmend eingestülpt, wobei das Injektionsmedium aus dem Behälter durch die Hohlnadel in das Gewebe gedrückt wird. Das obere Ende der Kanülenfassung (des Kanülensockels) ist so gestaltet, daß es genau in den Oberteil der Kapsel paßt und die Kapsel erforderlichenfalls vollständig entleert werden kann.

Da der Behälter vor Betätigung des Injektionsgerätes erfindungsgemäβ über dessen Oberseite hinausragt, ist der Zustand des Injektionsmediums vor Gebrauch einfach optisch kontrollierbar.

Für manche Injektionsmedien hat es sich als besonders zweckmäßig erwiesen, wenn der Behälter bzw. der Kapselvorsatz an seiner von der Kanüle durchstechbaren Seite zusätzlich mit einer Schutzfolie hermetisch verschlossen ist. Eine solche Folie kann ein- oder mehrschichtig aufgebaut sein, wobei sich eine Metallfolie allein oder kombiniert mit speziellen Kunststoff-Folien als sehr brauchbar erwiesen hat Infolge der außergewöhnlich guten Barrierewirkung einer dünnen und gut verformbaren Metallschicht (z. B. aus Aluminium) wird der Stoffaustausch zwischen der Füllung des Behälters und der Umgebungsluft um ein Vielfaches wirksamer unterbunden, als dies zur Zeit unter ausschließlicher Verwendung organischer Werkstoffe (z. B. von Elastomeren wie Silikongummi) möglich ist Die Lagerungsfähigkeit des Injektionsmediums wird somit stark verbessert. Dennoch wird die Verformbarkeit einzelner Lagen einer mehrschichtig aufgebauten Schutzfolie durch die Metallschicht nicht behindert, da die erwähnte Barrierewirkung bereits bei extrem dünnen, nur einige Mikrometer dicken Metallschichten zu beobachten ist, sofern letztere noch porenfrei sind.

An dem Sockel der Aufnahme gegenüber dem Fingerhebel ist zweckmäßig ein Gleitansatz vorgesehen, welcher in einem zweiten gewindegangförmigen Spalt in der Seitenwand des Gehäuses (der Konsole) geführt wird. Dieser zweite Spalt in der Seitenwand muss nicht nach außen offen sein.

Alternativ ist nur der gewindegangförmige Spalt zur Führung des Fingerhebels in der Seitenwand des Gehäuses vorgesehen und an dem Sockel der Aufnahme ist ein Außengewinde ausgebildet, welches in ein Innengewinde des Gehäuses mit gleicher Steigung wie der gewindegangförmige Spalt eingreift.

Der untere Bereich des Gehäuses ist vorteilhaft verstellbar - beispielsweise durch ein Schraubgewinde - mit der Bodenplatte verbunden; auf dem Gehäuse sind Stellmarken zur Einstellung der Stichtiefe angebracht und die Bodenplatte trägt eine korrespondierende Markierung. Bei Übereinstimmung von Stellmarken mit der Markierung können bei bestimmten Drehwinkeln relativ zum Gehäuse zusätzlich auch Einraststellen im Gewinde vorgesehen sein.

Die Einstellungen von Stichtiefe und Dosis können also mit taktilen Reizen - d. h. mit spürbarem Einrasten - verbunden sein.

Besonders zweckmäßig weist die Bodenplatte an ihrer Oberseite Fingermulden und an ihrer Unterseite Kontaktspitzen auf.

Die Fingermulden schließen miteinander meist einen spitzen Winkel ein; die Bodenplatte kann auf diese Weise mit zwei Fingern der linken oder rechten Hand gegen die Körperoberfläche gedrückt werden, um letztere vorzuspannen, eventuell vorgesehene Kontaktspitzen an der Unterseite der Bodenplatte wirksam werden zu lassen und um eine axiale Verdrehung des Injektionsgerätes bei Betätigung des Fingerhebels mit einem Finger der anderen Hand zu verhindern. Der Muldenrand zumindest einer Fingermulde kann seitlich hochgezogen sein, um einer eventuellen Taumelbewegung des Gehäuses beim Betätigen des Fingerhebels besser entgegenzuwirken und um das Injektionsgerät nach Gebrauch sicherer von der Körperoberfläche abnehmen zu können.

Die Kontaktspitzen können ihrer Aufgabe entsprechend hinsichtlich Größe und Gestalt verschieden ausgebildet sein. Kleinere Spitzen dienen üblicherweise zur besseren Fixierung des Injektionsgerätes über der ausgewählten Hautstelle (Haftspitzen), größere Spitzen - beispielsweise in der näheren Umgebung der Kanülenöffnung - sind zur Reizablenkung beim Einstechen der Nadel in die Haut gedacht, wodurch ein eventueller Stichschmerz relativ schwächer empfunden wird (Prinzip der "Skin stimulation").

Durch Andrücken der Bodenplatte mit der den Fingerhebel nicht betätigenden Hand wird wegen der so vorgespannten Haut die Läsion des Unterhautzellgewebes durch die eindringende Kanüle vermindert.

Das Verrutschen auf behaarter, nasser bzw. fettiger Haut oder auf dünner Bekleidung kann durch die feineren Haftspitzen auf der Unterseite der Bodenplatte vermieden werden und ein fallweiser Stichschmerz wird durch die erwähnten Reizspitzen relativiert

Fingerhebel bzw. Gleitansatz können vor Betätigung des Injektionsgerätes in die Fassung eines Montagespaltes knapp unterhalb des oberen Gehäuserandes oder in eine darunter liegende Rast im Anfangsbereich ihrer wendelförmigen Führungen geklemmt sein. Dieser kurze Anfangsbereich kann zunächst parallel zum Gehäuserand verlaufen oder in Verlängerung des gewindegangförmigen Führungsspaltes weiter nach oben geführt sein. Bei Ingebrauchnahme des Injektionsgerätes werden Fingerhebel bzw. Gleitansatz durch Überwindung eines geringen Widerstandes in die Führungen bewegt. Nach dem Zurückdrehen des Fingerhebels wird beispielsweise eine Noppe im Spalt überwunden und schließlich der Abschluss des Anfangsbereiches erreicht.

Es hat sich als günstig erwiesen, wenn eine annähernd ring- oder topfförmige Fingerrast an das Ende des Fingerhebels angeformt ist. Der Hebelarm kann zur Vermeidung einer Blockierung der Fingerrast durch auf der Bodenplatte befindliche Finger der anderen Hand hochgezogen sein. Die Hebelkraft kann durch einen kleineren Steigungswinkel des gewindegangförmigen Spalts oder durch radiale Verlängerung des Hebelarms erhöht werden.

Das Steuerelement, welches sich entweder an dem Sockel der Behälter- Aufnahme oder an der Kanülenhalterung befindet, weist gemäß einer Ausführungsform des erfindungsgemäßen Injektionsgerätes wenigstens eine periphere, kreisringförmige Ausnehmung auf und die Komplementäreinheit auf dem jeweils anderen der beiden genannten Bauteile umfasst zumindest ein Gleitstück mit einer geneigt zur Kanülenachse verlaufenden Gleitkante.

Abhängig von den geometrischen Gegebenheiten des Injektionsgerätes kann eine einzige Ausnehmung sowie eine einzige daran anschließende kreisförmige Gleitbahn ausreichend sein ("einspurig").

In vielen Fällen weist das Steuerelement aber zwei im Abstand voneinander befindliche, periphere kreisringförmige Ausnehmungen auf. Die Komplementäreinheit umfasst im letztgenannten Fall unbedingt zwei Gleitstücke, welche einander in Bezug auf die Kanülenachse annähernd gegenüberliegen und auf verschiedenen kreisförmigen Gleitbahnen des Steuerelementes bewegt werden ("zweispurige" Gleitstücke).

Zumindest eines der Gleitstücke weist eine geneigt zur Kanülenachse verlaufende Gleitkante auf.

Ein solches Gleitstück ist entsprechend dem Mantel eines Zylinders mit dem Durchmesser der Gleitbahn bzw. der Ausnehmung des Steuerelementes geformt. Rollt man das Gleitstück in eine Ebene auf, hat es im wesentlichen die Form eines rechtwinkeligen Dreieckes, dessen Hypotenuse die erwähnte "geneigte" Gleitkante darstellt Die anderen Winkel des rechtwinkeligen Dreieckes werden vom Anstieg des gewindegangförmigen Spaltes zur Führung des Fingerhebels sowie vom Radius der Gleitbahn bzw. der Ausnehmung festgelegt. Das zweite, gegenüberliegende Gleitstück kann analog zum ersten mit einer gleich langen - annähernd parallel zur Kanüle verlaufenden - Kathete geformt sein; da es sich aber auf einer Gleitbahn mit anderem Radius als das erste bewegt, müssen die beiden spitzen Winkel des Dreieckes von jenen des ersten Gleitstückes abweichen. Als zweites Gleitstück kann aber auch nur eine schmale Stütze von gleicher Länge wie die parallel zur Kanüle verlaufende Kathete des ersten Gleitstückes vorgesehen sein.

Nach einer anderen bevorzugten Ausführungsform ist das Steuerelement als zylindrisches, konzentrisch zur Kanülenachse angeordnetes Rohrstück mit zwei wendelförmigen offenen Spalten ausgebildet und die Komplementäreinheit weist zwei stabförmige, einen Ringspalt überbrückende Gleitstücke auf.

Als Gleitbahn dient dabei ein Teil des zwischen den wendelförmigen Spalten befindlichen, der Komplementäreinheit zugewendeten Randes des Rohrstückes.

Jede Gleitbahn des Steuerelementes weist - bezogen auf die Drehrichtung zum Absenken der Kanüle hinter der Ausgangsposition eines Gleitstückes der Komplementäreinheit - eine Falle auf. In eine solche Falle gelangen die Gleitstücke nach dem Zurückdrehen des Fingerhebels in den Anfangsbereich des gewindegangförmigen Spaltes. Die Falle kann z. B. als Senkfalle gestaltet sein; eine solche besteht lediglich aus einer Öffnung bzw. Vertiefung in einer Gleitbahn des Steuerelementes, aus welcher das Gleitstück nicht wieder heraus geschoben werden kann. Eine Keilfalle besteht aus einer keilförmig ansteigenden und sodann in einem rechten oder kleineren Winkel abfallenden Erhebung der Gleitbahn, über welche das Gleitstück nicht wieder zurückgeschoben werden kann. Senk- und Keilfalle können auch zu einer Falle kombiniert werden.

Nach dem Blockieren der Gleitstücke durch eine der genannten Fallen kann das Injektionsgerät nicht nochmals betätigt werden und es ist auch kein nachträgliches Auslaufen restlicher Injektionsflüssigkeit aus der Kanüle und/oder aus dem Injektionsmedien- Behälter möglich, da beim Zurückdrehen des Fingerhebels das obere Ende der Kanüle aus dem Behälter herausgezogen wird

Für den Einsatz unter speziellen Umgebungsvoraussetzungen, wie z. B. unter extremen Klimabedingungen oder extremer Fremd- bzw. Schadstoffbelastung der Umgebungsluft, wird bei einer weiteren Ausführungsform der gesamte Transportweg der injektionsflüssigkeit im Innern der Injektionsvorrichtung bis unmittelbar vor Herstellung des Hautkontakts speziell gegen den Außenraum abgedichtet Zu diesem Zweck ist an der Oberseite der Kanülenhalterung ein vor Gebrauch des Gerätes in einer zentralen Bohrung des Kolbenstopfens einer Karpule steckender Kanülensockel mit Dichtungswulst angeformt, an der Unterseite der Kanülenhalterung ist eine Kanülenkappe dichtend, aber entfernbar angebracht und die Riegel auf dem Stellring sind als Doppelriegel ausgeführt. Die Karpule ist in diesem Fall an ihrer von der Kanüle durchstechbaren Seite (Trennwand des Kolbenstopfens) nicht zusätzlich mit einer Schutzfolie verschlossen. Der Kanülensockel ist mit seinem Dichtungswulst in einen hülsenförmigen Einsatz des Kolbenstopfens oder direkt in die Bohrung des Kolbenstopfens geschoben. Vor Gebrauch des Injektionsgerätes muss lediglich die Kanülenkappe, z. B. mittels einer Schlaufe, aus der in diesem Falle größeren Kanülenöffnung des Gehäusebodens bzw. der Bodenplatte gezogen werden; die Druckscheibe wird in dieser Ausführungsform von dem erwähnten Doppelriegel gehalten.

Anstelle einer aus einem Stück gefertigten, an beiden Enden zugespitzten Kanüle ist es bisweilen zweckmäßig, wenn die Kanülenhalterung eine obere und eine untere einfach zugespitzte Kanüle enthält, deren Innenräume durch eine Verbindungsleitung miteinander verbunden sind. Der als Verbindungsleitung dienende Mittelteil ist beispielsweise als zentrale Bohrung in der Kanülenhalterung ausgeführt. Dieser Mittelteil hat ein größeres Lumen als die beiden einfach zugespitzten Enden der Hohlnadel, wodurch der Strömungswiderstand für das Injektionsmedium verringert wird.

Das erfindungsgemäße Einweg-Injektionsgerät besteht somit im wesentlichen aus sechs, überwiegend aus Kunststoff gefertigten und automationsgerecht nacheinander montierbaren Bauteilen sowie aus einem je nach Injektionsmedium (Medikament) variierbaren Behälter.

Die sechs Bauteile sind: Aufnahme für den mit Injektionsmedium gefüllten Behälter (z. B. eine Karpule oder eine Kapsel), Gehäuse (Konsole), elastisches Element wie z. B. eine Druckfeder, Kanülenhalterung (Nadelhalterung) und Bodenplatte. Schließlich ist noch ein Stellring über das Gehäuse geschoben.

Diese Bauteile weisen folgende Hauptmerkmale auf :
- Die Aufnahme umfasst den Sockel mit Fingerhebel, entweder das Steuerelement oder die Komplementäreinheit und gegebenenfalls einen Gleitansatz (Stutzen) bzw. alternativ ein Außengewinde. Die Aufnahme kann weiters eine zähelastische, die optische Kontrolle des Behälterinhalts ermöglichende Fassung aufweisen, in welche der Behälter eingesetzt wird ("snap in"). Eine solche Behälterfassung dient darüber hinaus als Bruchschutz für den Behälter.
- Das im wesentlichen zylindrische Gehäuse (Konsole) weist eine verstellbare Verbindung mit der Bodenplatte, die erwähnten wendelförmigen Führungen für den Fingerhebel und gewünschten falls für einen Gleitansatz (Stutzen) - wobei die Führung für den Gleitansatz die Gehäusewand nicht durchsetzen muss, d. h. nicht unbedingt ein offener Spalt sein muss - oder nur einen wendelförmigen offenen Spalt für den Fingerhebel und ein Innengewinde auf. Um den Fingerhebel vor Gebrauch der Injektionsvorrichtung aufzunehmen und festzuhalten, kann die früher bereits genannte Fassung im Montagespalt oder eine darunter liegende Rast vorgesehen sein. Auf der Gehäuseaußenseite befindet sich ein Einzug, um den offenen, auf dem Gehäuse drehbaren, elastischen Stellring in einer vorbestimmten Position zu halten; weiters sind Durchtrittsöffnungen (Fenster) für die zum Fixieren der Kanülenhalterung dienenden Riegel des Stellringes und an der Außenseite Blindfenster in Höhe der Durchtrittsöffnungen lediglich zum Einrasten dieser Riegel angeordnet Auf der Außenseite sind schließlich noch Stellmarken für Stichtiefe und Dosis angebracht. An der Innenseite befindet sich wenigstens eine parallel zur Kanülenachse verlaufende Führungsnut, welch letztere entsprechende Führungsleisten der Kanülenhalterung aufnimmt und eine Stufe, welche einen Anschlag für die Druckscheibe der abgesenkten Kanülenhalterung oberhalb des zusammengedrückten elastischen Elements (Druckfeder) bildet Der Gehäuseboden weist eine Kanülenöffnung auf.
- Das elastische Element kann beispielsweise eine im entlasteten Zustand bis nahe an die Oberkante des Gehäuses ragende, als Schraubenfeder ausgebildete Druckfeder aus Metall oder Kunststoff sein. Es dient zum Hochschieben der Nadelhalterung beim Zurückdrehen und/oder Loslassen des Fingerhebels.
- Die Kanülenhalterung (Nadelhalterung) umschließt die an beiden Enden zugespitzte Kanüle (Injektionsnadel), deren obere Spitze nicht ausstanzend ("non coding") ausgebildet ist (oder zwei einfach zugespitzte Kanülen, deren Innenräume durch eine Leitung - z. B. eine zentrale Bohrung in der Kanülenhaterung - miteinander verbunden sind). Die Kanülenhalterung verjüngt sich zur oberen Kanülenspitze hin in vielen Fällen zu einer Kanülenfassung und schließlich zu einem Kanülensockel, der z. B. in die Bohrung oder in einen hülsenartigen Einsatz in der Bohrung des verschiebbaren Kolbenstopfens einer Karpule passt Das obere Ende des Kanülensockels kann kuppel- bzw. halbkugelartig gestaltet sein, bei speziellen Ausführungsformen ist zusätzlich ein Dichtungswulst angeformt Mit einem solchen, zum injektionsmedienbehälter hin abgerundeten Kanülensockel ist eine gegebenenfalls vorgesehene Schutzfolie besser durchdringbar. Ist eine Kapsel mit Kapselvorsatz als Behälter vorgesehen, so bildet die Kanülenfassung bzw. der Kanülensockel zweckmäßig auch das Gegenstück zum Oberteil der Kapsel. Der Kanülenhalterung bzw. der Kanülenfassung mit Sockel kommt somit in jedem Fall zugleich auch die Funktion eines Verdrängers zu.

Die Kanalenhalterung umfasst an ihrem unteren Teil weiters eine Druckscheibe (Federplatte), eine oder mehrere Führungsleisten (in die Führungsnuten an der Innenseite des Gehäuses eingreifend), ein Steuerelement oder eine Komplementäreinheit Gemeinsam mit den entsprechenden, weiter oben beschriebenen Gegenstücken auf der Aufnahme wird mit den zuletzt genannten Bauteilen die Zwangssteuerung des Injektionsgerätes bewerkstelligt.
- Die Bodenplatte weist an ihrer Oberseite Fingermulden und an ihrer Unterseite gewünschten falls Kontaktspitzen verschiedener Gestalt und Länge auf. Die Verbindung der Bodenplatte mit dem Gehäuse kann z.B. durch ein Schraubgewinde erfolgen, welch letzteres mit Einrastungen bei bestimmten Drehwinkeln relativ zum Gehäuse zwecks Einstellung der Stichtiefe versehen ist, wodurch das Einrasten auch taktil und/oder akustisch wahrgenommen werden kann. Eine Markierung am Rand der Bodenplatte zeigt dabei auf entsprechende Stellmarken auf dem Gehäuse. Eine zentrale Kanülenöffnung fluchtet mit der Kanolenöffnung im Gehäuseboden.
- Als Stellring dient ein elastischer, offener Ring mit strukturierter, rutschsicherer Außenseite (z. B. Riefelung), welcher in meist zwei Ausnehmungen jeweils eine federnde Zunge mit einem in zwei Richtungen keilförmig abgeschrägten, zunächst auf dem oberen Rand der Druckscheibe aufliegenden Riegel oder mit einem diesen Rand beidseitig umfassenden Doppelriegel zur Fixierung der Druckscheibe in einer Ausgangsposition und zum Einrasten in Blindfenstern an der Außenseite des Gehäuses, welches auch taktil und/oder akustisch wahrnehmbar ist Eine zusätzlich vorgesehene Hebelsperre kann hoch- oder tiefgestellt sein; auf dem Stellring befindet sich in diesem Fall auch eine mit den Dosis- Stellmarken auf der Gehäusewand zusammenwirkende Markierung.

Das erfindungsgemäß umgesetzte technische Konzept bietet folgende Vorteile gegenüber herkömmlichen vorgefüllten Einmalspritzen :
- Das Injektionsmedium kann sowohl in starren, zylindrischen, einseitig offenen Glas- oder Kunststoffgefäßen mit beweglichem Kolbenstopfen, beispielsweise Karpulen, als auch in deformierbaren Kunststoffbehältern, beispielsweise Kapseln, abgefüllt vorliegen.
- Diese Behälter können gegen Umgebungseinflüsse zusätzlich mittels einer Schutzfolie hermetisch abgeschlossen sein.
- Der Zustand der Injektionsmedien vor Gebrauch ist einfach optisch kontrollierbar.
- Der Behälter ist gegen Beschädigung durch Schlag oder Fall abgeschirmt
- Die Stichtiefe kann durch Veränderung der Distanz zwischen dem Gehäuseboden und der Bodenplatte voreingestellt werden.
- Die Maximaldosis kann durch Blockieren des Fingerhebels in bestimmter Höhe verringert werden.
- Die Einstellung von Stichtiefe und Dosis kann auch taktil (spürbares Einrasten) und/oder akustisch (Klicken) wahrgenommen werden
- Durch Andrücken der Bodenplatte mit der den Fingerhebel nicht betätigenden Hand wird die Haut vorgespannt und die Nadel streng koaxial geführt und die Läsion des Unterhautzellgewebes durch die eindringende Kanüle weitgehend vermindert.
- Eventuelles Verrutschen des Injektionsgerätes auf nasser, behaarter bzw. fettiger Haut oder auf dünner Bekleidung kann durch Haftspitzen vermieden werden.
- Ein fallweiser Stichschmerz kann durch Reizspitzen relativiert werden.
- Die Kanüle ist für den Patienten während des gesamten Injektionsvorgangs nicht sichtbar
- Die Kanüle wird zum Abschluss des Injektionsvorgangs wieder vollständig in das Gehäuse zurückgezogen und zugleich gegen nochmaliges Austreten gesichert.
- Das Hantieren mit ungeschützter Kanüle vor und/oder nach dem Gebrauch des Injektionsgerätes entfällt.
- Das Innere der Kanüle bleibt bis zum Erreichen der eingestellten Stichtiefe trocken - für die Verabreichung bestimmter Injektionslösungen eine unbedingte Voraussetzung.
- Der Entleerungsdruck und die Dosiergeschwindigkeit bzw. die Dauer des Injektionsvorgangs sind über die Steigung der Hebelführung und/oder die Hebellänge des Fingerhebels gut steuerbar.
- Der sichere Gebrauch ist auch bei gesundheitlichen Beeinträchtigungen (z.B. Stress, Nadelphobie, Tremor, Sehschwäche) möglich.
- Der gesamte Injektionsvorgang, i.e. die Abfolge von Einstechen in das Hautgewebe, Durchstechen einer Behälterwandstelle, Entleeren des Behälters, Zurückziehen der Kanüle aus der Einstichstelle und Sicherung vor nochmaligem Austritt der Nadel wird beim Betätigen des Fingerhebels durch das Zusammenspiel der beiden Konstruktionselemente "Komplementäreinheit" (Gegenstück) und "Steuerelement" zwangsgesteuert; die Gefahr einer fehlerhaften Funktion oder Anwendung ist damit (auch bei extremen Umgebungstemperaturen) stark eingeschränkt.
- Wegen der einfachen und sicheren Handhabung des Injektionsgeräts kann vielfach auf die Assistenz von medizinischem Fachpersonal - insbesondere bei der Selbstbehandlung von Patienten oder bei der Versorgung von Patienten im Not- bzw. Katastrophenfall - verzichtet werden.
- Es entsteht kein gefährlicher Abfall.

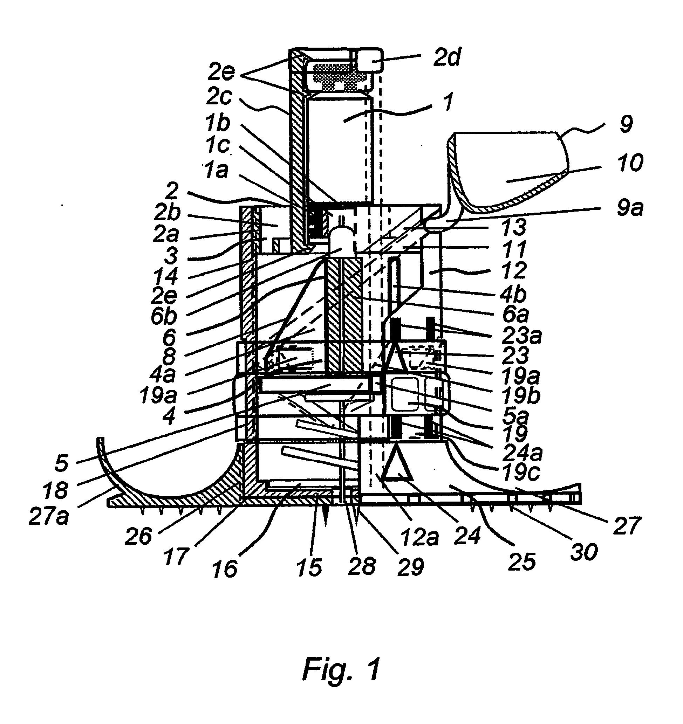

In den Fig. 1 bis 4 wird das erfindungsgemäße Injektionsgerät und dessen Funktion am Beispiel von vier Ausführungsformen noch näher erläutert.

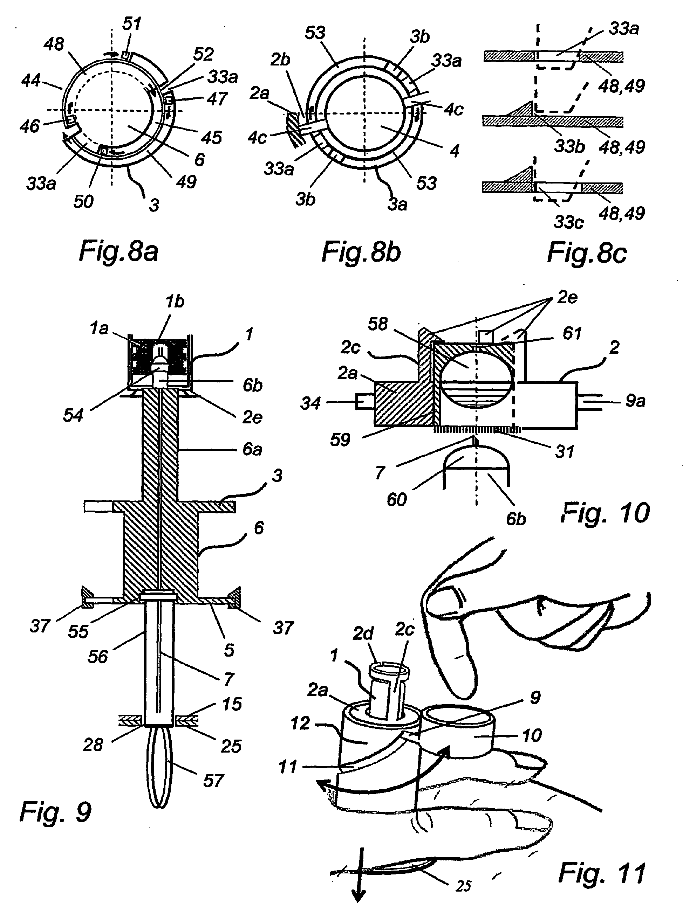

Fig. 1 zeigt ein Injektionsgerät - teilweise im Schnitt - mit einer Karpule 1 als Behälter. Der Sockel 2a der Behälter- Aufnahme 2 mit Ringspalt 2b trägt ein zweispuriges Steuerelement 3 mit zwei kreisringförmigen Ausnehmungen (in Fig. 8a genauer vorgestellt). Die Karpule 1 ist in einer Behälterfassung 2c gehaltert, welch letztere gleichfalls einen Teil der Aufnahme 2 bildet Dargestellt ist eine Behälterfassung 2c, die aus einer einzigen Stütze mit oben angeformtem, offenem Schutzring 2d besteht Auch mehrere Stützen sind möglich, der Inhalt der Karpule muß jedoch leicht optisch überprüft werden können. Die Karpule 1 wird von ringförmigen, abgeschrägten Klammem 2e an ihrem unteren Rand und im Bereich ihrer oberen, meist metallischen Verschlusskappe gehaltert Beim Zusammenbau des Injektionsgerätes wird die Karpule 1 einfach von oben her bis zum Einrasten in die Behälterfassung 2c eingeschoben. An ihrer Unterseite ist die Karpule 1 mit einem Kolbenstopfen 1a verschlossen. Der Kolbenstopfen 1a ist an seiner Peripherie mit Dichtringen versehen; in eine zentralen Bohrung des Stopfens, welche bis an seine Trennwand 1b reicht, ist ein versteifender, hülsenförmiger Einsatz 1c eingebracht.

Die mit dem Steuerelement 3 zusammenwirkende Komplementäreinheit 4 besteht aus zwei Gleitstücken 4a, 4b, welche auf der Druckscheibe 5 der Kanülenhalterung 6 angeordnet sind. Das "äußere" Gleitstück 4a, welches von der Achse der Kanüle 7 weiter entfernt ist als das "innere" Gleitstück 4b, weist eine geneigt zu dieser Achse verlaufende Gleitkante 8 auf. Das "innere" Gleitstück ist lediglich eine dünne Stütze 4b mit annähernd quadratischem Querschnitt und von gleicher Länge wie die parallel zur Kanüle verlaufende Kante des äußeren Gleitstückes. Beide Gleitstücke sind genügend schmal ausgeführt, um durch die Ausnehmungen im Steuerelement 3 durchtreten zu können. Um eine Drehung der Kanülenhalterung 6 zu verhindern, ist der Rand der Druckscheibe 5 mit wenigstens einer Führungsleiste 5a versehen, welche jeweils in einer parallel zur Kanülenachse an der Wandinnenseite des Gehäuses 12 vorgesehenen Führungsnut 12a gleitet An die Druckscheibe 5 der Kanülenhalterung 6 schließt eine Kanülenfässung 6a an, welche sich zur oberen Kanülenspitze hin zu einem oben abgerundeten Kanülensockel 6b weiter verjüngt.

Der Aufnahmesockel 2a ist mit einem Fingerhebel 9, dessen Hebelarm 9a hochgezogen ist und an dessen Ende sich eine Fingerrast 10 befindet, verbunden. Der Hebelarm 9a ragt durch einen gewindegangförmigen offenen Führungsspalt 11 in der Seitenwand des Gehäuses 12. Die Begrenzungslinien dieses Spaltes sind gerade verlaufend dargestellt, wenngleich der Schraubengang in der Seitenansicht schwach S-förmig gekrümmt erscheinen würde. Der vor der Bildebene liegende Teil des Spaltes ist strichliert gezeichnet. Der Anstieg des Spaltes 11 entspricht jenem eines Außengewindes 13 an der Aufnahme 2, welches in ein Innengewinde 14 des Gehäuses 12 eingreift. Zwischen Druckscheibe 5 und dem Boden 15 des Gehäuses 12 ist eine konische Druckfeder 16 angeordnet, wobei die Druckscheibe 5 in Richtung Gehäuseboden 15 nur bis zu einer ringförmigen Stufe 17 bewegt werden kann. Beim Zusammendrücken der Feder 16 finden deren Windungen Platz in dem durch Stufe 17 ausgesparten Raum.

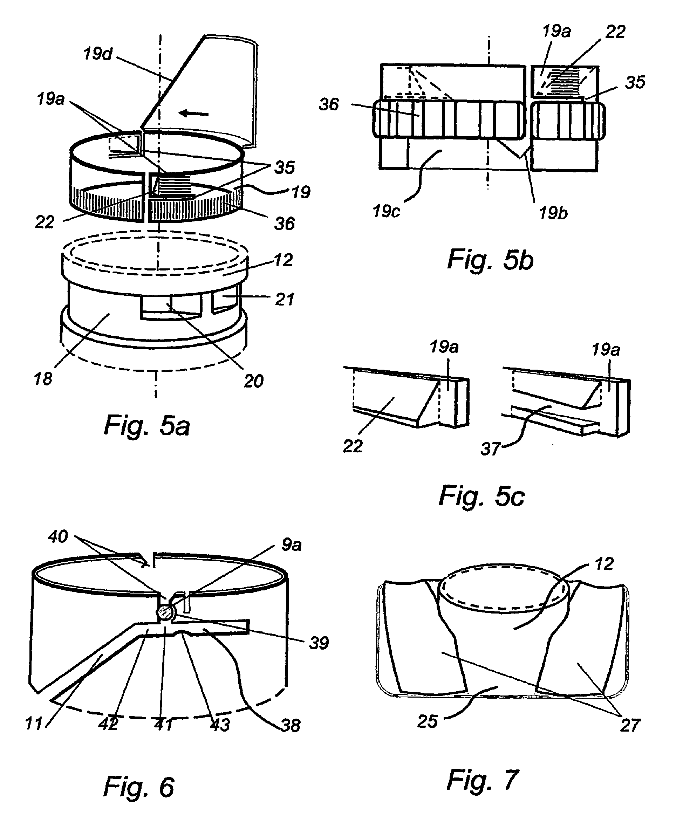

An der Außenseite des Gehäuses 12 ist ein ringförmiger Einzug 18 vorgesehen, in dem der offene Stellring 19 drehbar gelagert ist. Stellring 19 weist eine geriefelte Außenseite, zwei elastisch federnde Zungen 19a am oberen Rand, eine vom oberen Rand ausgehende tiefgestellte Hebelsperre 19b und eine Ausnehmung 19c im Bereich des unteren Randes auf. Auf der Innenseite der federnden Zungen 19a befindet sich jeweils ein in zwei Richtungen abgeschrägter Riegel. Vor Betätigung des Injektionsgerätes ragen die beiden Riegel durch entsprechende Fenster in der Gehäusewand und halten die Druckscheibe 5 der Kanülenhalterung 6 gegen den Druck des elastischen Elementes 16 **fest.** Ein Stellring dieser Art ist in Fig. 5b noch detailreicher dargestellt Ein Abschnitt der zylindrischen Seitenwand des Gehäuses 12 mit Einzug 18, einem Fenster 20 und Blindfenster 21 ist der Fig. 5a schematisch zu entnehmen. Der beschriebene Riegel ist in der linken Darstellung von Fig. 5c vergrößert gezeigt und mit 22 bezeichnet Im oberen Bereich des Stellringes ist weiters eine Markierung 23 angebracht, die mit Dosis- Stellmarken 23a auf der Gehäusewand- Außenseite **zusammenwirkt.** Durch Ausnehmung 19c können auch Stellmarken 24a für die Einstichtiefe auf der Gehäusewand-Außenseite erkannt werden. Eine dreieckige Markierung 24 auf der Bodenplatte 25 zeigt beim Drehen der Bodenplatte auf bestimmte Stellmarken 24a, wodurch gleichzeitig beabsichtigte Einstichtiefen der Kanüle in das Gewebe festgelegt werden. Die Bodenplatte 25 weist eine Schraubverbindung 26 mit dem unteren Bereich des Gehäuses 12 auf. Das Schraubgewinde kann zusätzlich mit den Stellmarken 24a korrespondierende Einrastungen enthalten. In die Oberseite der Bodenplatte 25 sind zwei Fingermulden 27 eingeformt Der Rand 27a einer dieser Mulden ist hochgezogen. Eine zentrale Kanülenöffnung 28 liegt genau unter einer entsprechenden Öffnung im Gehäuseboden 15. In der nächsten Umgebung der Kanülenöffnung 28 sind Reizspitzen 29 an der Unterseite der Bodenplatte 25 vorgesehen und über die verbleibende Fläche der Unterseite verteilt befinden sich Haftspitzen 30. Der Außenrand der Bodenplatte 25 ist wie Stellring 19 zur Erhöhung der Griffsicherheit mit Riefen versehen.

Die Vorgangsweise bei der Verwendung des efindungsgemäßen Injektionsgerätes wird im folgenden Text beschrieben:

Das Injektionsgerät wird einer Sterilverpackung entnommen und der Inhalt der Karpule 1 optisch auf etwaige Verfärbungen oder Trübungen kontrolliert. Das zur Injektion vorgesehene Hautareal wird nötigenfalls gereinigt bzw. desinfiziert.

Mit der einen Hand wird der obere Teil des Injektionsgerätes einschließlich des Fingerhebels 9 festgehalten und mit der anderen Hand die Bodenplatte 25 mit Markierung 24 im Uhrzeigersinn (von oben gesehen) bis zum Einrasten der Schraubverbindung 26 bei einer bestimmten Stellmarke 24a gedreht, wobei der Abstand zwischen Gehäuse(Konsolen-)boden 15 und Bodenplatte 25 vergrößert wird; um denselben Betrag weniger weit tritt bei gleicher Endstellung des Fingerhebels 9 die untere Spitze der Kanüle 7 aus der Nadelöffnung 28 aus.

Mit der letztgenannten Hand werden daraufhin die beiden Riegel 22 durch Drehen des Stellrings 19 gegen den Uhrzeigersinn (von oben gesehen) aus den Fenstern 20 in der Gehäusewand herausgezogen und rasten zunächst im ersten Blindfenster 21 ein. Dadurch wird der Rand der Druckscheibe 5 freigegeben und letztere schnellt unter Einwirkung der Druckfeder 16 ein kurzes Stück aufwärts ("Aktivierung"), bis die Spitzen der Gleitstücke 4a, 4b der Komplementäreinheit 4 auf die kreisringförmigen Gleitbahnen des Steuerelements 3 (s. Fig. 8a) treffen; dabei wird der Kanülensockel 6b weiter in den Einsatz 1c des Kolbenstopfens 1a hinein geschoben und die obere Kanülenspitze gelangt bis an die Trennwand 1b des Kolbenstopfens 1a. Durch Weiterdrehen des Stellrings 19 zu den nächsten Blindfenstern kann die Hebelsperre 19b an unterschiedliche Stellen vor den gewindegangförmigen Führungsspalt 11 gesetzt werden und verkürzt somit die anschließende Abwärtsbewegung des Fingerhebeis 9. Der Vorschub des Kolbenstopfens1a in die Karpule 1 wird proportional dazu begrenzt und die injizierte Dosis vermindert

Die Bodenplatte 25 wird mit zwei, in den Fingermulden 27 der Bodenplatte aufliegenden Fingern der einen, z. B. der linken Hand, gegen die vorgesehene Körperoberfläche gedrückt wobei die Kontaktspitzen 29, 30 an der Unterseite der Bodenplatte wirksam werden. Durch den festen Andruck der starren Bodenplatte wird zugleich die Hautoberfläche vorgespannt sowie die Lage der eindringenden Kanüle gegenüber dem Gewebe bis zum Erreichen der Stichtiefe stabilisiert. Selbst bei unruhiger Betätigung des Fingerhebels 9 mit einem Finger der anderen Hand kann die Hohlnadel 7 ihre Lage und Richtung nicht verändern.

Durch Drehen des Fingerhebels 9 mit einem in der Fingemast 10 befindlichen Finger der freien, z. B. der rechten Hand im Uhrzeigersinn (von oben betrachtet) wird der Fingerhebel zuerst aus einer Fassung im Montagespalt oder aus einer darunter liegenden Rast im Anfangsbereich der Hebelführung (genauer in Fig. 6) und dann in einer schraubenförmigen Drehung durch den Spalt 11 abwärts bewegt wobei die während der Aktivierung des Injektionsgerätes um ein kurzes Stück angehobene untere Kanülenspitze wieder zur Nadelöffnung 28 in der Bodenplatte 25 gelangt Die Spitzen der Gleitstücke 4a, 4b der Komplementäreinheit 4 gleiten dabei vorerst auf zwei kreisförmigen, konzentrischen Gleitbahnen des Steuerelementes 3 und die Kanülenhalterung 6 mit Druckscheibe 5 wird in Richtung Behälterboden 15 gedrückt. Da die Führungsleisten 5a auf dem Rand der Druckscheibe bei dieser Bewegung in den Führungsnuten 12a gleiten, wird eine Drehung der Kanülenhalterung 6 verhindert. Die untere Kanülenspitze tritt aus der Nadelöffnung 28 in der Bodenplatte aus und dringt in das Hautgewebe ein.

Beim weiteren Abwärtsdrehen des Fingerhebels 9 gelangen die Spitzen der Gleitstücke 4a, 4b ganz knapp vor dem Kontakt der Druckscheibe 5 mit der Stufe 17 über die Kanten der an die Gleitbahnen anschließenden, konzentrischen Ausnehmungen im Steuerelement 3, wodurch die Absenkung der Kanülenhalterung 6 kurz unterbrochen wird und zugleich die Trennwand 1b des Kolbenstopfens 1a mit der oberen Kanülenspitze in Kontakt kommt Beim Auftreffen der Druckscheibe 5 auf der Innenstufe 17 der Konsole ist die vorgesehenen Einstichtiefe im Gewebe erreicht und durch Weiterdrehen des Fingerhebels 9 durchsticht die obere Kanülenspitze nunmehr die Trennwand 1b und die Kanülenfassung 6a schiebt den Kolbenstopfen 1a in die Karpule 1. Das Injektionsmedium wird dabei verdrängt und strömt durch die Kanüle in die Injektionsstelle. Die Abwärtsbewegung der Aufnahme 2 mit Karpule 1 und damit der Grad der Entleerung, d. h. die abgegebene Dosis an Injektionsmedium, wird durch die eingestellte Position der Hebelsperre 19b des Stettrings 19 begrenzt.

Sobald der Fingerhebel 9 beim Abwärtsdrehen auf den bleibenden Widerstand der Hebelsperre 19b stößt bzw. das Ende des Führungsspaltes 11 erreicht ist, wird der Fingerhebel 9 unter Aufrechterhaltung des Andrucks der Bodenplatte 25 an die Körperoberfläche zügig zurückgedreht, bis der Hebelarm 9a im Anfangsbereich des Führungsspaltes 11 abermals auf bleibenden Widerstand stößt Dabei werden zuerst die Gleitstücke 4a, 4b über zumindest eine geneigte Gleitkante 8 wieder zurück auf die Gleitbahnen des Steuerelementes 3 gezogen, sodann wird die Kanüle 7 gemeinsam mit der Kanülenhalterung 6 angehoben und schließlich aus dem Hautgewebe zurück in das Gehäuse (die Konsole) 12 gezogen. Am Ende der Gleitbahnen des Steuerelementes 3 gelangt zumindest die Spitze eines Gleitstückes in eine Falle gemäß Fig. 8c, wodurch ein abermaliges Abwärtsdrehen des Hebelarms 9a und der Wiederaustritt der unteren Kanülenspitze aus der Bodenplatte 25 blockiert wird.

Bei Verwendung eines elastischen Elements mit ausreichend hoher Federkraft erfolgt der gesamte Rückstellvorgang selbsttätig, sobald der Fingerhebel losgelassen wird.

Erst nachdem der Fingerhebel 9 im Anfangsbereich des Führungsspaltes 11 in einer Falle fixiert ist, wird der Andruck der beiden Finger in den Fingermulden 27 der Bodenplatte 25 gegen die Körperoberfläche aufgehoben und das Injektionsgerät abgenommen. Das gebrauchte Gerät kann gefahrlos entsorgt werden.

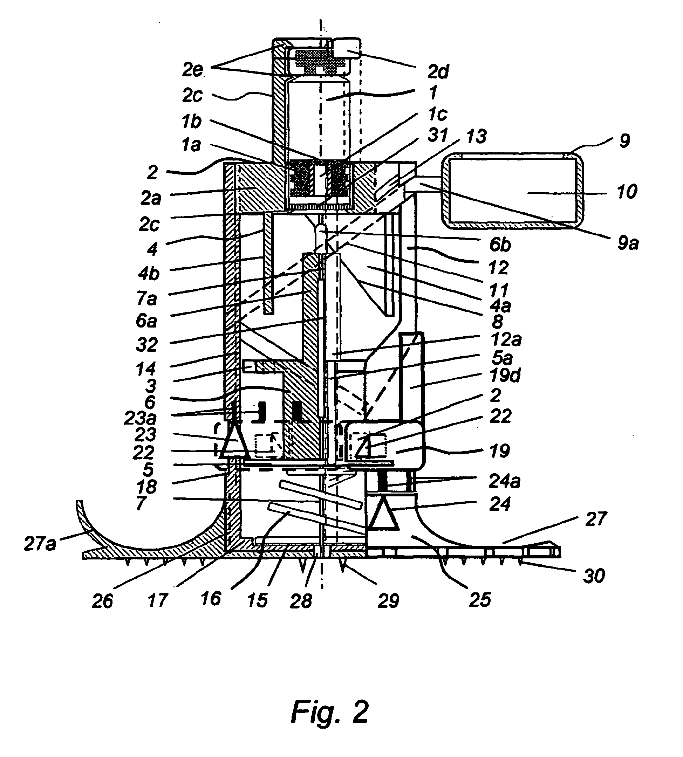

Der Fig. 2 ist gleichfalls ein teilweise im Schnitt dargestelltes Injektionsgerät mit einer Karpule 1 als Behälter zu entnehmen. Das - analog Fig. 1 zweispurig ausgeführte - Steuerelement 3 ist hier ein Teil der Kanülenhalterung 6. Der Sockel 2a der Aufnahme 2 trägt die Komplementäreinheit 4, welche wie in Fig. 1 zwei Gleitstücke 4a, 4b umfasst. Mit dem Sockel 2a ist ein Fingerhebel 9 verbunden, dessen Hebelarm 9a abweichend von der in Fig. 1 gezeigten Ausführungsform nicht hochgezogen ist und an dessen Ende eine topfförmige, nach oben offene Fingerrast 10 angeformt ist

Kärpule 1 ist an ihrer von der einfach zugespitzten oberen Kanüle 7a durchstechbaren Seite zusätzlich mit einer Schutzfolie 31 hermetisch verschlossen. Die Kanülenhalterung 6 enthält in ihrer zentralen Kanütenfässung 6a die obere (7a) und die untere (7b) einfach zugespitzte Kanüle, deren Innenräume durch die annähernd in der Achse der Kanülenfassung 6a verlaufende Leitung bzw. Bohrung 32 verbunden sind.

Stellring 19 weist eine hochgesetzte (hochgestellte) Hebeisperre 19d auf; er ist der oberen Zeichnung von Fig. 5a in perspektivischer Darstellung genauer zu entnehmen.

Die weiteren Bauteile finden ihre Entsprechungen in analogen Elementen der Fig. 1 und wurden daher mit den gleichen Bezugszeichen versehen.

Die Spitze der oberen Kanüle 7a durchdringt im Laufe der Verwendung des Injektionsgerätes nicht nur die Trennwand 1b des Kolbenstopfens 1a, sondern gemeinsam mit ihrem oben abgerundeten Sockel 6b vorher auch die Schutzfolie 31.

Der großteils zwangsgesteuerte Ablauf der einzelnen Injektionsschritte wurde bereits im Zusammenhang mit Fig. 1 genauer erläutert.

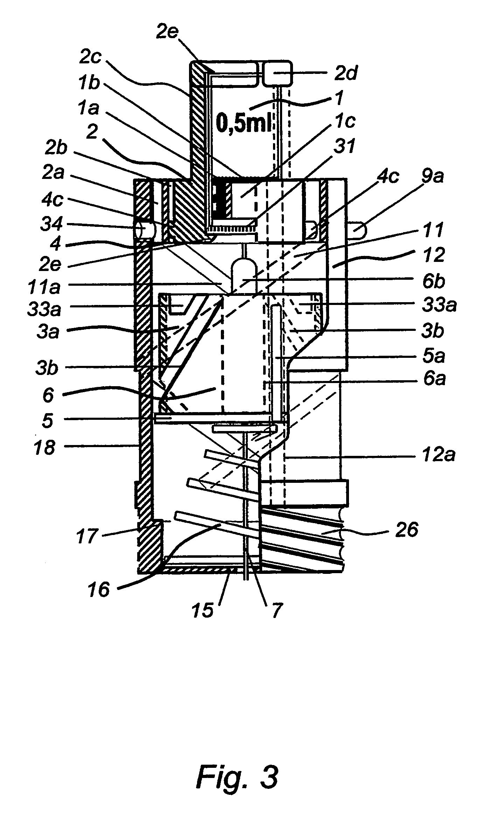

In der in Fig. 3 gezeigten Ausführungsform wurden entsprechenden Bauteilen wieder die Bezugsziffern gemäß den Fig. 1 und 2 zugeordnet. Im Unterschied zu den Fig. 1 und 2 sind Fingerrast 10, Stellring 19, Stellmarken 23a, 24a und Bodenplatte 25 in dieser Darstellung nicht mehr enthalten, um insbesondere die abweichend ausgebildeten Bauteile - nämlich Steuerelement und Komplementäreinheit - sowie deren Zusammenspiel zu erläutern. Als Behälter 1 ist ein zylindrisches, einseitig offenes Gefäß mit Kolbenstopfen 1a dargestellt

Nach Aktivierung des Gerätes durch Drehen des Stellringes mit tiefgestellter Hebeisperre (s. Fig. 1, Fig. 5b) schnellt die Druckscheibe 5 einschließlich Kanolenhalterung 6 und Steuerelement 3a unter Einwirkung der Druckfeder 16 aufwärts, bis die Gleitbahnen des Steuerelementes 3a auf die stabförmigen Gleitstücke 4c der Komplementäreinheit 4 treffen. Das Steuerelement 3a hat im wesentlichen die Form eines Rohrstückes mit zwei wendelförmigen, offenen Spalten 3b. Als Gleitbahnen dienen Abschnitte auf der Oberkante des Rohrstückes zwischen den Spalten 3b und den beiden Senkfallen 33a (vgl. dazu auch Fig. 8b und Fig. 8c). Die stabförmigen Gleitstücke 4c der Komplementäreinheit 4 überbrücken den im Sockel 2a der Aufnahme 2 vorgesehenen Ringspalt 2b.

Die obere Spitze der Kanüle 7 durchdringt im Verlauf der Aktivierung die Schutzfolie 31 an der Unterseite der Karpule 1 und gelangt im Kolbeneinsatz 1c bis an die Trennwand 1 b.

Durch Weiterdrehen des Stellrings 19 zu den nächsten Blindfenstern 21 (Fig. 5a) kann die tiefgestellte Hebelsperre 19b (Fig. 5b) an unterschiedlichen Stellen vor den Führungsspalt 11 gesetzt werden und verkürzt diesen entsprechend; proportional dazu wird die injizierte Dosis vermindert.

Anstelle eines Außengewindes an der Aufnahme 2 bzw. an deren Sockel 2a, welches in ein Innengewinde des Gehäuses 12 eingreift, ist ein Gleitansatz 34 an dem Sockel 2a vorgesehen. Dieser Gleitansatz 34 wird in einem zweiten gewindegangförmigen Spalt 11a in der Seitenwand des Gehäuses 12 geführt. Gemäß Fig. 3 muss der Spalt 11a die Seitenwand nicht vollständig durchsetzen - er muss somit nicht "offen" ausgeführt sein. Der offene Führungsspalt 11 ist an jenen Stellen, wo er oberhalb der Bildebene verläuft, strichliert dargestellt Der Ganganstieg des Spaltes 11 muss jenem des zweiten Spaltes 11a gleichen.

Durch Betätigung des Fingerhebels wird der Hebelarm 9a zunächst aus einer Fassung im Montagespalt oder aus einer darunter liegenden Rast im Anfangsbereich der Hebelführung (Fig. 6) gedrückt und dann gemeinsam mit Aufnahme 2 und Gleitansatz 34 in einer schraubenförmigen Drehung in den Führungsspalten 11 bzw. 11a abwärts bewegt. Dabei gleiten die beiden Gleitstücke 4c der Komplementäreinheit 4 auf den Gleitbahnen des nicht um die Kanülenachse drehbaren, zylindrischen Steuerelementes 3a, welches so wie Druckscheibe 5 einen Teil der Kanülenhalterung 6 darstellt Die untere Spitze der Kanüle 7 dringt in das Hautgewebe ein.

Beim weiteren Abwärtsdrehen des Fingerhebels werden ab einem Sicherheitsabstand - d. h. kurz vor dem Kontakt von Druckscheibe 5 mit Stufe 17 - die beiden Gleitstücke 4c über die Kanten der wendelförmigen offenen Spalten 3b des Steuerelementes 3a geschoben, wodurch die Absenkung der Kanülenhalterung 6 kurz unterbrochen wird und zugleich die obere Spitze der Kanüle 7 die Trennwand 1b des Kolbenstopfens 1a erreicht. Nach dem Auftreffen der Druckscheibe 5 auf der Innenstufe 17 des Gehäuses 12 ist auch die vorgesehene Einstichtiefe in das Gewebe erreicht und die obere Spitze der Kanüle 7 durchsticht die Trennwand 1b. Die Kanülenfassung 6a schiebt den Kolbenstopfen 1a in die Karpule 1, deren Inhalt dabei durch die Kanüle entleert wird. Die Abwärtsbewegung der Karpule 1 und damit der Grad ihrer Entleerung, i. e. die abgegebene Dosis an Injektionsmedium, wird durch die Stellung der Hebeisperre des Stellrings begrenzt.

Sobald der Fingerhebel beim Abwärtsdrehen auf den bleibenden Widerstand der Hebeisperre stößt, wird er unter Aufrechterhaltung des Andrucks der Bodenplatte an die Körperoberfläche zügig in entgegen gesetzter Richtung wieder zurückgedreht. Dabei werden zuerst die beiden Gleitstücke 4c der Komplementäreinheit 4 durch die Spalten 3b des Steuerelementes 3a wieder zurück auf die Gleitbahnen des Steuerelementes 3a gezogen, sodann wird die Kanülenhalterung 6 mitsamt der Kanüle 7 unter Einwirkung des elastischen Elementes 16 angehoben und schließlich wird die untere Spitze der Hohlnadel 7 aus dem Hautgewebe zurück in das Gehäuse 12 gezogen. Am Ende der Gleitbahnen gelangt zumindest ein Gleitstück 4c, gegebenenfalls über einen keilförmig ansteigenden Abschnitt, in die Öffnung einer Falle 33a, wodurch eine abermalige Betätigung des Fingerhebels und somit ein Wiederaustritt der unteren Kanülenspitze aus der Kanülenöffnung in der Bodenplatte blockiert ist

Bei Verwendung eines elastischen Elements mit ausreichend hoher Federkraft erfolgt der gesamte Rückstellvorgang selbsttätig, sobald der Fingerhebel losgelassen wird.

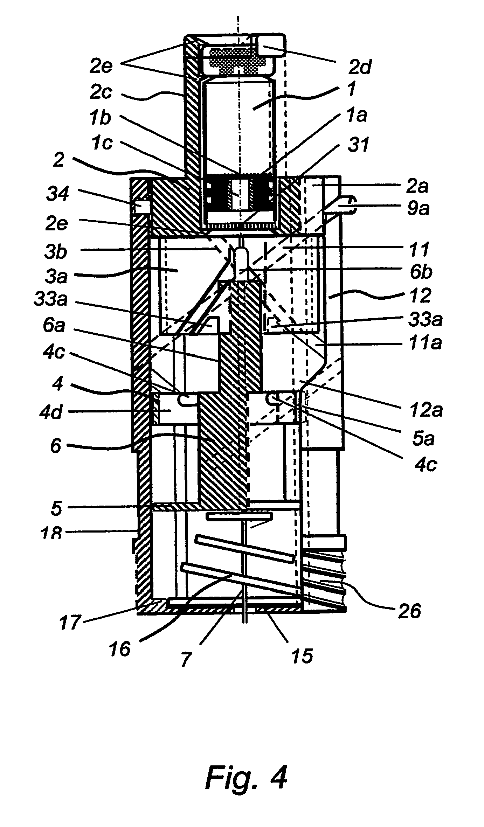

Das Einweg- Injektionsgerät gemäß Fig. 4 unterscheidet sich von der aus Fig. 3 ersichtlichen Ausführungsform insbesondere hinsichtlich der Anordnung von Steuerelement 3a und Komplementäreinheit 4. Entsprechende Bauteile sind mit den gleichen Bezugsziffern wie in Fig. 3 bezeichnet. Der Sockel 2a der Behälter- Aufnahme 2 trägt das konzentrisch zur Kanülenachse angeordnete, mit dem Sockel 2a drehbare Steuerelement 3a.

Die nicht um die Kanülenachse drehbare Kanülenhalterung 6 weist als Komplementäreinheit 4 zwei stabförmige Gleitstücke 4c, welche einen Ringspalt 4d überbrücken, auf.

Die Gesamthöhe des Gerätes nach Fig. 4 ist geringfügig größer als jene des in Fig. 3 gezeigten, der Ablauf der Injektion ist dem in Zusammenhang mit Fig. 3 erläuterten analog.

Die Fig. 5a bis 11 zeigen beispielhaft weitere Merkmale bestimmter Ausführungsformen.

Der in Fig. 5a perspektivisch dargestellte, offene Stellring 19 mit hochgesetzter Hebelsperre 19d ist beispielsweise für das in Fig. 2 wiedergegebene Injektionsgerät geeignet. Er wird vor dem Einsetzen der Aufnahme 2 von jener Seite des Gehäuses 12 her, welche dem Gehäuseboden 15 gegenüber liegt, über den Einzug 18 an der Wand des Gehäuses 12 geschoben. Ein Abschnitt der Seitenwand des Gehäuses 12, auf welchem der Einzug 18, ein Fenster 20 und ein Blindfenster 21 zu erkennen sind, ist unter dem Stellring 19 schematisch dargestellt Die beiden in zwei Richtungen keilförmig abgeschrägten Riegel 22 (vergrößert in Fig. 5c, linke Darstellung) auf der Innenseite der elastisch federnden, entlang der Schlitze 35 des Stellringes ausschwenkbaren Zungen 19a treten durch die Fenster 20 des Gehäuses 12 in dessen Inneres. Die aus der gleichen Richtung wie der Stellring 19 gegen den Druck einer Druckfeder eingeschobene Druckscheibe (5 in Fig. 2) der Kanülenhalterung wird mittels der Riegel 22 in ihrer Lage festhalten.

Bei Gebrauch wird der Stellring 19 mit zwei Fingern am Riefelrand 36 gefasst und in Richtung zum ersten Blindfester 21 verdreht, wobei die Riegel 22 über die abgeschrägte Kante des Fensters 20 nach außen gleiten und die Druckscheibe freigeben, sodass letztere unter dem Druck der Druckfeder nach oben schnellen kann. Wird der Stellring weitergedreht, bis der Riegel im nächsten Blindfenster einrastet, dann schiebt sich die vom Stellring 19 nach oben ragende ("hochgesetzte") Hebeisperre 19d ein Stück weit über das untere Ende des Führungsspaltes (11 in Fig. 2), blockiert so die vollständige Abwärtsbewegung des Fingerhebels, begrenzt damit das aus dem Injektionsmedien- Behälter verdrängte Volumen und vermindert auf diese Weise die injizierte Dosis.

Der Stellring nach Fig. 5b ist beispielsweise für die in den Fig. 1 oder 3 wiedergegebenen Ausführungsformen geeignet Die Funktionsweise gleicht jener des Stellrings gemäß Fig. 5a, wegen der insaesamt niedrigeren Konstruktion der Injektionsgeräte nach Fig. 1 oder 3 geht aber eine "tiefgesetzte" Hebeisperre 19b vom oberen Rand des Ringes aus und setzt sich in den Ring fort Ausnehmung 19c gibt die Sicht auf Stellmarken (24a in Fig. 1) frei.

Fig. 5c sind zwei Möglichkeiten zur Ausbildung der an den elastisch federnden Zungen eines Stellringes befindlichen Riegel zu entnehmen. Ein in zwei Richtungen keilförmig abgeschrägter Riegel 22 ist für Injektionsgeräte gemäß den Fig. 1 bis 4 geeignet, genauso aber auch ein Doppelriegel 37.

Soll der gesamte Transportweg der Injektionsflüssigkeit im Innern eines erfindungsgemäßen Injektionsgerätes bis unmittelbar vor Herstellung des Hautkontakts besonders gut gegen den Außenraum abgedichtet sein, so kann es das in Fig. 9 schematisch vorgestellte Aussehen haben. In diesem Fall ist ein Doppelriegel 37 unbedingt vorzusehen, um die Kanülenkappe unmittelbar vor Gebrauch des Gerätes abziehen zu können, ohne die Kanülenhalterung gleichzeitig in Richtung Bodenplatte zu bewegen.

Fig. 6 zeigt eine Ausführungsmöglichkeit für den Anfangsbereich 38 eines zur Führung des Hebelarmes 9a bzw. zur Führung eines gegenüberliegenden Gleitansatzes 34 (entsprechend den Fig. 2 und 4, nicht eingezeichnet) vorgesehenen gewindegangförmigen Spaltes 11 bei einem erfindungsgemäßen Injektionsgerät Dieser kurze Anfangsbereich 38 im oberen Bereich des Gehäuses 12 kann zunächst im wesentlichen parallel zum oberen Rand des Gehäuses 12 verlaufen. Im Ausgangszustand sind der Hebelarm 9a des Fingerhebels und ein eventueller Gleitansatz (34) jeweils in eine Fassung 39 im Montagespalt 40 oder in eine darunter liegende Rast 41 mit Rückhalt 42 geklemmt, von wo aus sie bei Ingebrauchnahme des Gerätes nach Überwindung eines geringen Widerstandes in den gewindegangförmigen Spalt 11 (bzw. 11a) bewegt werden. Das Zurückdrehen des Fingerhebels erfolgt über die im Anfangsbereich 38 vorgesehene Noppe 43 hinweg bis zum bleibenden Widerstand am Abschluss des Anfangsbereiches 38.

Auch bei einem Injektionsgerät mit einem Gewinde (13 in den Fig. 1 und 2) auf dem Sockel der Behälter- Aufnahme kann der in Verlängerung des gewindegangförmigen Spalts schräg nach oben geführte Anfangsbereich in gleicher Weise ausgestaltet sein

In Fig. 7 sind die Fingermulden 27 auf der Bodenplatte 25 vereinfacht dargestellt. Die Mulden schließen miteinander einen spitzen Winkel ein; die Bodenplatte kann auf diese Weise mit zwei Fingern der linken oder rechten Hand gegen die Körperoberfläche gedrückt werden, um letztere vorzuspannen, eventuell vorgesehene Kontaktspitzen an der Unterseite der Bodenplatte wirksam werden zu lassen und um eine axiale Verdrehung des injektionsgerätes bei Betätigung des Fingerhebels mit einem Finger der anderen Hand zu verhindern. Der Muldenrand zumindest einer Fingermulde kann, wie in Fig. 1 gezeigt, seitlich hochgezogen sein, um einer eventuellen Taumelbewegung des Gehäuses 12 beim Betätigen des Fingerhebels besser entgegenzuwirken und um das injektionsgerät nach Gebrauch sicherer von der Körperoberfläche abnehmen zu können.

In Fig. 8a ist ein in den injektionsgeräten gemäß den Fig. 1 und 2 verwendetes "zweispuriges" Steuerelement 3 - in Richtung der Kanülenachse gesehen - veranschaulicht Das mit dem Sockel 2a der Aufnahme 2 (Fig. 1) oder mit der Kanülenhalterung 6 (Fig. 2) verbundene Steuerelement 3 weist zwei im Abstand voneinander befindliche, periphere kreisringförmige Ausnehmungen 44, 45 auf. Die einander diametral gegenüberliegenden Spitzen eines inneren (4b in den Fig.1 und 2) bzw. eines äußeren Gleitstücks (4a in Fig.1 und in Fig.2) befinden sich zunächst in ihren Ausgangspositionen 46 bzw. 47 auf der inneren (48) bzw. der äußeren Gleitbahn 49 des zweispurigen Steuerelements 3. Die Gleitstücke gelangen bei Betätigung des injektionsgerätes allmählich in Richtung der Pfeile von den Gleitbahnen in die daran anschließenden Ausnehmungen 44, 45 und schließlich zu den Endpositionen 50, 51. Nach dem abschließende Zurückdrehen des Fingerhebels in den Anfangsbereich der Hebelführung werden die Gleitstückspitzen bis in die vor deren Ausgangspositionen liegenden Fallen, die beispielsweise als Senkfallen 33a ausgebildet sind, geführt. Für den Fall, dass periphere Ausnehmungen (44, 45) direkt aneinandergrenzen oder dass eine Senkfalle 33a unmittelbar neben eine Ausnehmung 45 zu liegen kommt, kann ein festigkeitserhöhender Steg 52 vorgesehen sein. Anstelle von Senkfallen (33a) sind z. B. auch Keilfallen (33b in Fig. 8c) oder eine Kombination der beiden (33c in Fig. 8c) möglich

Fig. 8b ist ein zylindrisches, mit einer Kanülenhalterung (6) verbundenes "einspuriges" Steuerelement (Rohrstück) 3a mit zwei wendelförmigen offenen Spalten 3b in Draufsicht zu entnehmen. Eine geeignete Komplementäreinheit (4) weist gemäß Fig. 3 zwei stabförmige Gleitstücke 4c auf, welche einen Ringspalt 2b im Sockel 2a der stark vereinfacht dargestellten Aufnahme 2 überbrücken. Die stabförmigen Gleitstücke 4c gelangen aus ihren der Fig. 8b entnehmbaren Ausgangspositionen in Pfeilrichtung über eine den oberen Rand des Steuerelements 3a bildende Gleitbahn 53 in die Spalten 3b des Steuerelements 3a; nach Erreichen der Endpositionen unmittelbar über der Druckscheibe (5 in Fig. 3) werden sie beim Zurückdrehen des Fingerhebels in die zwischen den Ausgangspositionen und den wendelförmigen Spalten 3b angeordneten Fallen auf dem oberen Rand des Rohrstückes 3a, die beispielsweise als Senkfallen 33a ausgebildet sind, geführt.

Ein Steuerelement 3a nach Fig. 8b kann auch mit dem Sockel 2a der Aufnahme 2 verbunden sein (Fig. 4). Die Komplementäreinheit 4 bildet in diesem Fall eine Einheit mit der Kanülenhalterung 6. Die stabförmigen Gleitstücke 4c erreichen ihre Endpositionen unmittelbar unterhalb des Sockels 2a und gelangen schließlich in Fallen (z. B. Senkfallen 33a) auf dem unteren Rand des Rohrstückes 3a.

Verschiedene Ausführungen von Fallen für strichliert angedeutete Gleitstücke (4a, 4b gemäß Fig. 1 und 2) sind in Fig. 8c schematisch dargestellt Die Senkfalle 33a besteht lediglich aus einer Öffnung in einer Gleitbahn 48, 49 eines Steuerelementes 3 (Fig. 8a), aus welcher das Gleitstück nicht wieder heraus geschoben werden kann. Die Keilfalle 33b besteht aus einer anfangs keilförmig ansteigenden und sodann in einem rechten oder kleineren Winkel zur Gleitbahn abfallenden Erhebung, über welche das Gleitstück nicht wieder zurückgeschoben werden kann. Senk- und Keilfalle können auch zu einer Falle 33c kombiniert werden (Fig. 8c, untere Darstellung). Fallenformen mit gleicher Wirkung gelangen auch auf Gleitbahnen 53 im Zusammenhang mit Steuerelementen 3a gemäß Fig. 8b zum Einsatz. Eine reine Senkfalle 33a kann in letzterem Fall als trapezförmige Vertiefung in einer Gleitbahn 53 ausgebildet sein (vgl. Fig. 3 und 4).

Nach dem Blockieren der Gleitstücke durch eine der beschriebenen Fallen kann das Injektionsgerät nicht nochmals betätigt werden und es ist auch kein nachträgliches Auslaufen restlichen Injektionsmediums aus Kanüle und/oder Behälter möglich, da dies die Kapillarkräfte in der Kanüle verhindern und beim Zurückdrehen des Fingerhebels auch die obere Kanülenspitze aus der Trennwand des elastischen Kolbenstopfens bzw. aus der verformbaren Kapselwand herausgezogen wird und sich die Durchtrittsöffnungen wieder weitgehend selbst verschließen.

Wie bereits weiter oben erläutert, ist für den Einsatz unter außergewöhnlichen Umgebungsvoraussetzungen eine spezielle Variante des erfindungsgemäßen Injektionsgerätes vorgesehen. Fig. 9 zeigt schematisch und beispielhaft die Gestaltung wesentlicher Bauteile eines solchen Gerätes. An der Oberseite der Kanülenhalterung 6 bzw. der Kanülenfassung 6a ist ein Kanülensockel 6b mit Dichtungswulst 54 angeformt Ein von der Kanülenhalterung 6 getragenes Steuerelement ist mit 3 bezeichnet. Vor Gebrauch des Gerätes steckt der Kanülensockel 6b dichtend in einer zentralen Bohrung des Kolbenstopfens 1a einer Karpule 1. An der Unterseite der Kanülenhalterung 6 - ungefähr in Höhe der Druckscheibe 5 - ist konzentrisch zur Kanüle 7 eine profilierte Ausnehmung 55 vorgesehen, in welche eine Kanülenkappe 56 dichtend eingesetzt ist. Die Kanülenkappe 56 reicht bis in die vergrößerte Kanülenöffnung 28 im Gehäuseboden 15 bzw. in der Bodenplatte 25. Vom Stellring sind nur die beiden Doppelriegel 37 (Fig. 5c), welche die Druckscheibe 5 vor Verwendung des Gerätes in Position halten, dargestellt. Die Kanülenkappe 56 kann unmittelbar vor Gebrauch des Gerätes mittels einer Schlaufe 57 aus der profilierten Ausnehmung 55 gezogen werden. Der weitere Injektionsablauf entspricht der bereits im Zusammenhang mit den Fig. 1 bis 4 dargelegten Funktionsweise.

In Fig. 10 ist eine Aufnahme 2 zu erkennen, deren Sockel 2a eine Behälter- Fassung 2c, einen Hebelarm 9a und einen Gleitansatz 34 aufweist Drei Klammem 2e der Fassung 2c sind sichtbar. Eine deformierbare Kunststoffkapsel 58, beispielsweise in Form eines Rotationsellipsoids mit zwecks besserer Deformierbarkeit kreisförmigen Vertiefungen im verformbaren Teil, befindet sich in einem Kapselvorsatz 59, welcher an seiner Unterseite noch mit einer Schutzfolie 31 hermetisch verschlossen ist. Der Kapselvorsatz 59 ist in einen Klemmsitz des Aufnahme- Sockels 2a eingebracht Die obere Spitze der Kanüle 7 befindet sich vor Aktivierung des Injektionsgerätes knapp unter der Schutzfolie 31; die Spitze der Hohlnadel 7 ragt aus der abgerundeten Kuppe 60 des Kanülensockels 6b bzw. der Kanülenfassung. Die Kuppe 60 ist dem Oberteil der Kapsel 58 genau angeformt, sodass letztere erforderlichenfalls vollständig entleert werden kann. Die Kanülenspitze kann dabei bis in die Bohrung 61 in der oberen Begrenzung des Kapselvorsatzes 59 vordringen.

Fig. 11 zeigt ein stark schematisiertes, erfindungsgemäßes Injektionsgerät mit einer Karpule 1 als Injektionsmedien- Behälter im Gebrauch. Die Bodenplatte 25 wird mit zwei Fingern der einen Hand gegen die Körperoberfläche gedrückt und der Fingerhebel 9 mit einem Finger der anderen Hand in dem gewindegangförmigen Spalt 11 bis zum bleibenden Widerstand abwärts und danach wieder bis zum bleibenden Widerstand zurückgedreht; anschließend wird der Druck gegen die Körperoberfläche aufgehoben und die Injektionsvorrichtung von der Körperoberfläche abgenommen. -

## Patentansprüche

1. Einweg- Injektionsgerät mit einem Behälter für Injektionsmedien, welcher von einem Ende einer an beiden Enden zugespitzten Kanüle durchstechbar ist, und mit einer Einrichtung zur irreversiblen Fixierung der Kanüle in einem Gehäuse nach Gebrauch des Injektionsgerätes, **dadurch gekennzeichnet, dass**.
- der Behälter (1) vor Befätigung des Injektions gerätes über dessen Oberseite hinansragt und einer Aufnahme (2) gehaltert ist, welche in dem Gehäuse (12) drehbar und gleichzeitig in Richtung der Kanülenachse verschiebbar ist,
- in der Seitenwand des Gehäuses (12) zumindest ein gewindegangförmiger Spalt (11) zur Führung eines mit dem Sockel (2a) der Aufnahme (2) verbundenen Fingerhebels (9) vorgesehen ist, wobei der Sockel (2a) entweder ein Steuerelement (3) oder eine Komplementäreinheit (4) für das Steuerelement(3) trägt,
- im Inneren des Gehäuses (12) koaxial und unterhalb der Aufnahme (2) eine in Richtung der Kanülenlachse verschiebbare, aber nicht um die Kanülenachse drehbare Kanülenhalterung (6) angeordnet ist, welche Kanülenhalterung (6) entweder eine mit dem Steuerelement (3) auf dem Sockel (2a) zusammenwirkende Komplementäreinheit (4) oder ein zu der Komplementäreinheit (4) auf dem Sockel (2a) korrespondierendes Steuerelement (3) aufweist,
- am unteren Ende der Kanülenhalterung (6) eine Druckscheibe (5) ausgebildet ist, die unter dem ständigen Druck eines zwischen dem Boden (15) des Gehäuses (12) und der Druckscheibe (5) angeordneten elastischen Elementes (16) steht,
- der untere Bereich des Gehäuses (12) mit einer Bodenplatte (25) verbunden ist, welche wie der Gehäuseboden (15) eine zentrale Kanülenöffnung (28) aufweist, und dass
- um die Seitenwand des Gehäuses (12) ein offener Stellring (19) mit wenigstens einem von außen durch zumindest ein Fenster (20) in der Seitenwand eingreifenden keilförmigen Riegel (22, 37) vorgesehen ist, welcher Riegel (22, 37) die Druckscheibe (5) und damit die Kanülenhalterung (6) vor Gebrauch des Gerätes in einer Ausgangsposition hält (Fig. 5a, Fig. 5b, Fig. 5c)

2. Einweg-Injektionsgerät nach Anspruch 1, **dadurch gekennzeichnet, dass** der Stellring (19) zusätzlich eine Hebelsperre (19b, 19d) aufweist (Fig. 1, Fig. 2, Fig. 5a, Fig. 5b)).

3. Einweg-Injektionsgerät nach den Ansprüchen 1 bis 2, **dadurch gekennzeichnet, dass** an dem Sockel (2a) der Aufnahme (2) gegenüber dem Fingerhebel (9) ein Gleitansatz (34) vorgesehen ist, welcher in einem zweiten gewindegangförmigen Spalt (11a) in der Seitenwand des Gehäuses (12) geführt wird. (Fig. 3, Fig. 4, Fig. 10).

4. Einweg-Injektionsgerät nach den Ansprüchen 1 bis 2 **dadurch gekennzeichnet, dass** an dem Sockel (2a) der Aufnahme (2) ein Außengewinde (13) ausgebildet ist, welches in ein Innengewinde des Gehäuses (12) mit gleicher Steigung wie der gewindegangförmige Spalt (11) zur Führung des Fingerhebels (9) eingreift. (Fig. 1, Fig. 2)

5. Einweg-Injektionsgerät nach den Ansprüchen 1 bis 4, **dadurch gekennzeichnet, dass** das Steuerelement (3) wenigstens eine periphere, kreisringförmige Ausnehmung (44, 45) aufweist und die Komplementäreinheit (4) zumindest ein Gleitstück (4a) mit einer geneigt zur Kanülenachse verlaufenden Gleitkante (8) umfasst (Fig. 1, Fig. 2, Fig. 8a).

6. Einweg-Injektionsgerät nach den Ansprüchen 1 bis 4 **dadurch gekennzeichnet, dass** das Steuerelement (3a) als zylindrisches, konzentrisch zur Kanülenachse angeordnetes Rohrstück mit zwei wendelförmigen offenen Spalten (3b) ausgebildet ist und die Komplementäreinheit (4) zwei stabförmige Gleitstücke (4c), welche einen Ringspalt (2b, 4d) überbrücken, aufweist (Fig. 3, Fig. 4, Fig. 8b).

7. Einweg-Injektionsgerät nach den Ansprüchen 1 bis 6, **dadurch gekennzeichnet, dass** die Bodenplatte (25) an ihrer Oberseite Fingermulden (27) und an ihrer Unterseite Kontaktspitzen (29, 30) aufweist und verstellbar mit dem unteren Bereich des Gehäuses (12) verbunden ist (Fig. 1, Fig. 2, Fig. 7).

8. Einweg-Injektionsgerät nach den Ansprüchen 1 bis 7 **dadurch gekennzeichnet, dass** das Gehäuse (12) durch ein Schraubgewinde (26) mit der Bodenplatte (25) verbunden ist, auf dem Gehäuse (12) Stellmarken (24a) zur Einstellung der Stichtiefe angebracht sind und die Bodenplatte (25) eine korrespondierende Markierung (24) trägt. (Fig. 1, Fig. 2).

9. Einweg-Injektionsgerät nach den Ansprüchen 1 bis 8, **dadurch gekennzeichnet, dass** eine Karpule (1) deren verschiebbarer, flexibler Kolbenstopfen (1a) zur oberen Kanülenspitze gerichtet ist, als Behälter eingesetzt wird. (Fig. 1 bis Fig. 4)

10. Einweg-Injektionsgerät nach Anspruch 9 **dadurch gekennzeichnet, dass** an der Oberseite der Kanülenhalterung (6) ein vor Gebrauch des Gerätes in einer zentralen Bohrung des Kolbenstopfens (1a) steckender Kanülensockel (6b) mit Dichtungswulst (54) angeformt ist, dass an der Unterseite der Kanülenhalterung (6) eine Kanülenkappe (56) dichtend, aber entfernbar angebracht ist und dass die Riegel auf dem Stellring (19) als Doppelriegel (37) ausgeführt sind. (Fig. 5c, Fig. 9)

11. Einweg-Injektionsgerät nach den Ansprüchen 1 bis 8 **dadurch gekennzeichnet, dass** eine Kapsel (58) mit Kapselvorsatz (59) als Behälter eingesetzt wird. (Fig. 10)

12. Einweg-Injektionsgerät nach den Ansprüchen 1 bis 11, **dadurch gekennzeichnet, dass** jede Gleitbahn (48, 49, 53) des Steuerelementes (3, 3a) - bezogen auf die Drehrichtung zum Absenken der Kanüle (7) hinter der Ausgangsposition (46, 47) eines Gleitstückes (4a, 4b, 4c) der Komplementäreinheit (4) eine Falle (33a, 33b, 33c) aufweist (Fig. 3, Fig. 4, Fig. 8a, Fig. 8b, Fig. 8c).

13. Einweg-Injektionsgerät nach den Ansprüchen 1 bis 12, **dadurch gekennzeichnet, dass** der Behälter (1, 58) an seiner von der Kanüle (7) durchstechbaren Seite zusätzlich mit einer Schutzfolie (31) hermetisch verschlossen ist. (Fig. 2, Fig. 3, Fig. 4, Fig. 10).

14. Einweg-Injektionsgerät nach den Ansprüchen 1 bis 13, **dadurch gekennzeichnet, dass** eine topfförmige Fingerrast (10) an das Ende des Fingerhebels (9) angeformt ist. (Fig. 1, Fig. 2, Fig. 11).

15. Einweg-Injektionsgerät nach den Ansprüchen 1 bis 14 **dadurch gekennzeichnet, dass** die Kanülenhalterung (6) eine obere (7a) und eine untere (7b) einfach zugespitzte Kanüle enthält, deren Innenräume durch eine Verbindungsleitung (32) miteinander verbunden sind. (Fig. 2).

## Claims

1. Disposable injection apparatus, featuring a container for injection media that may be penetrated by one end of a double-pointed injection needle and an equipment to irreversibly fixate the needle within a case after use, **characterized in that**
- the container (1) that protrudes over the top of the apparatus prior to use and is held by a fixture (2) that can be rotated and moved in the direction of the needle axis within the case (12) at the same time,
- at least one thread turn-shaped slit (11) that leads a finger lever (9), which is connected to the base (2a) of the fixture (2) is provided in the side wall of the case (12), whereas the base (2a) carries either a control element (3) or a complementary unit (4) for the control element (3),
- a needle holder (6) that can be moved in the direction of the needle axis but cannot be rotated around the needle axis, is located coaxially and below the fixture (2) inside the case (12), which needle holder (6) features either a complementary unit (4) to work together with the control element (3) on the base (2a) or a control element (3) to correspond to the complementary unit (4) on the base (2a),
- a pressure disc (5)is formed at the lower end of the needle holder (6), which is under constant pressure from an elastic element (16) that is located between the pressure disc (5) and the bottom (15) of the case (12),
- the lower part of the case (12) is connected with a baseplate (25) that shows a central opening (28) for the injection needle like the bottom (15) of the case (12) does, and
- an open adjusting collar (19) that carries at least one tapered latch (22, 37), which can be inserted into at least one window (20) in the case wall is provided around the case (12), to keep the pressure disc (5), and therefore the needle holder (6), in a starting position before use (Fig. 5a, Fig. 5b, Fig. 5c).

2. Disposable injection apparatus according to claim 1, **characterized in that** the adjusting collar (19) additionally shows a lever blocker (19b, 19d). (Fig. 1, Fig. 2, Fig. 5a, Fig. 5b)

3. Disposable injection apparatus according to claims 1 and 2, **characterized in that** a lug (34) is provided at the base (2a) of the fixture (2), located vis-à-vis the finger lever (9), to be moved along a second thread turn-shaped slit (11a) in the side wall of the case (12) (Fig. 3, Fig. 4, Fig. 10).

4. Disposable injection apparatus according to claims 1 and 2, **characterized in that** an external screw thread (13) is formed on the base (2a) of the fixture (2) to engage with a screw thread at the inner side of the case (12), showing the same slope as the thread turn-formed slit (11) that leads the finger lever (9). (Fig. 1, Fig. 2)

5. Disposable injection apparatus according to claims 1 to 4, **characterized in that** the control element (3) shows at least one peripheral, annular recess (44, 45), and the complementary unit (4) comprises at least one sliding lug (4a) with an edge (8) sloped towards the axis of the needle (Fig. 1, Fig. 2, Fig. 8a).

6. Disposable injection apparatus according to claims 1 to 4, **characterized in that** the control element (3a) is formed as a cylindric tube, positioned concentrically around the axis of the needle, showing two coiled slits (3b), and the complementary unit (4) shows two rod-shaped lugs (4c) that bridge an annular gap (2b, 4d) (Fig. 3, Fig. 4, Fig. 8b).

7. Disposable injection apparatus according to claims 1 to 6, **characterized in that** the baseplate (25) shows recesses (27) for the fingers on its upper side, shows spikes (29, 30) on its lower side, and is, though adjustable, connected to the lower part of the case (12). (Fig. 1, Fig. 2, Fig. 7).

8. Disposable injection apparatus according to claims 1 to 7, **characterized in that** the case (12) is connected to the baseplate (25) via a screw thread (26), the case (12) shows setting marks (24a) to adjust the injection depth, and the baseplate (25) shows corresponding markings (24). (Fig. 1, Fig. 2).

9. Disposable injection apparatus according to claims 1 to 8, **characterized in that** a carpule (1), whose moveable, flexible gasket (1a) is oriented towards the upper tip of the needle, is used as a container (Fig. 1 to Fig. 4).

10. Disposable injection apparatus according to claim 9, **characterized in that** a socket (6b) for the needle with a sealing ridge (54) is formed on the upper side of the needle holder (6) and is nested in a central bore in the gasket (1 a) prior to use, an airtight but removable cap (56) for the needle is fixed to the bottom of the needle holder (6), and the latches on the adjusting collar (19) are implemented as double latches (37) (Fig. 5c, Fig. 9)

11. Disposable injection apparatus according to claims 1 to 8, **characterized in that** a capsule (58) with an adaptor (59) is used as a container (Fig. 10).

12. Disposable injection apparatus according to claims 1 to 11, **characterized in that** every slipway (48, 49, 53) of the control element (3, 3a), regarding the sense of direction to lower the needle (7), shows a catch (33a, 33b, 33c) behind the starting position (46, 47) of a sliding lug (4a, 4b, 4c) of the complementary unit (4). (Fig.3, Fig. 4, Fig 8a, Fig. 8b, Fig. 8c).

13. Disposable injection apparatus according to claims 1 to 12, **characterized in that** the container (1, 58) shows an additional protective foil (31) that hermetically seals the side of the container to be punctured by the needle (7) (Fig. 2, Fig. 3, Fig. 4, Fig. 10).

14. Disposable injection apparatus according to claims 1 to 13, **characterized in that** the finger lever (9) shows a cup-shaped finger rest (10) (Fig. 1, Fig. 2, Fig. 11).

15. Disposable injection apparatus according to claims 1 to 14, **characterized in that** the needle holder (6) holds an upper (7a) and a lower (7b) single-pointed injection needle, whose interior spaces are connected through a connecting duct (32) (Fig. 2).

## Revendications

1. Appareil d'injection à usage unique avec réservoir pour la substance d'injection, réservoir pouvant être percé par l'extrémité d'une canule aux deux extrémités pointues, et avec un dispositif rendant définitive la fixation de la canule dans un boîtier après utilisation de l'appareil d'injection, et ayant les caractéristiques suivantes :
- avant utilisation de l'appareil d'injection, le réservoir (1) dépassant au-dessus de la partie supérieure de l'appareil est placé dans un élément de réception (2) pouvant tourner dans le boîtier (12) et pouvant en même temps se déplacer dans le sens de l'axe de la canule,
- la paroi latérale du boîtier (12) comporte au moins un espace spiralé (11) destiné au guidage d'un poussoir (9) relié au socle (2a) de l'élément de réception (2), dispositif dans le cadre duquel le socle (2a) porte soit un élément de commande (3) soit une unité complémentaire (4) pour l'élément de commande (3),
- un élément de retenue (6) de canule, pouvant se déplacer dans le sens de l'axe de la canule sous l'élément de réception (2) mais ne pouvant pas tourner autour de cet axe, est disposé à l'intérieur du boîtier (12) coaxialement, sachant que l'élément de retenue de canule (6) présente soit une unité complémentaire (4) qui coopère avec l'élément de commande (3) sur le socle (2a), soit un élément de commande (3) qui correspond à l'unité complémentaire (4) sur le socle (2a),
- à l'extrémité inférieure de l'élément de retenue de canule (6) est monté un disque de compression (5) qui subit en continu l'action d'une pression exercée par un élément élastique (16) placé entre le fond (15) du boîtier (12) et le disque de compression (5),
- la partie inférieure du boîtier (12) est reliée à une plaque de fond (25) qui présente comme le fond du boîtier (15) une ouverture de canule centrale (28),
- une bague d'arrêt ouverte (19) est prévue au niveau de la paroi latérale du boîtier (12) avec au moins un élément de verrouillage cunéiforme (22, 37) venant s'imbriquer de l'extérieur à travers au moins une fenêtre (20) dans la paroi latérale, dispositif dans le cadre duquel l'élément de verrouillage (22, 37) maintient le disque de compression (5) et ainsi l'élément de retenue de canule (6) en position initiale avant l'utilisation de l'appareil. (Fig. 5a, Fig. 5b, Fig. 5c).

2. L'appareil d'injection à usage unique selon la revendication 1 présente la caractéristique que la bague d'arrêt (19) dispose en plus d'un levier de verrouillage (19b, 19d). (Fig. 1, Fig. 2, Fig. 5a, Fig. 5b).

3. L'appareil d'injection à usage unique selon les revendications 1 à 2 présente la caractéristique qu'un épaulement coulissant (34) est prévu au niveau du socle (2a) de l'élément de réception (2) en face du poussoir (9), épaulement qui est dirigé dans un deuxième espace spiralé (11a) au niveau de la paroi latérale du boîtier (12). (Fig. 3, Fig. 4, Fig. 10).

4. L'appareil d'injection à usage unique selon les revendications 1 à 2 présente la caractéristique qu'un filetage externe (13) est présent au niveau du socle (2a) de l'élément de réception (2), filetage qui intervient pour guider le poussoir (9) dans un filetage interne du boîtier (12) avec la même inclinaison que l'espace spiralé (11). (Fig. 1, Fig. 2).

5. L'appareil d'injection à usage unique selon les revendications 1 à 4 présente la caractéristique que l'élément de commande (3) présente au moins une cavité périphérique circulaire (44, 45) et que l'unité complémentaire (4) comprend au moins une pièce coulissante (4a) avec un bord coulissant (8) incliné sur l'axe de la canule. (Fig. 1, Fig. 2, Fig. 8a).

6. L'appareil d'injection à usage unique selon les revendications 1 à 4 présente la caractéristique que l'élément de commande (3a) est monté comme un tuyau cylindrique, disposé de façon concentrique par rapport à l'axe de la canule avec deux espaces ouverts spiralés (3b), et que l'unité complémentaire (4) présente deux pièces coulissantes en forme de bâtons (4c) pontant un espace circulaire (2b, 4d). (Fig. 3, Fig. 4, Fig. 8b).

7. L'appareil d'injection à usage unique selon les revendications 1 à 6 présente la caractéristique que la plaque de fond (25) dispose sur sa face supérieure de cavités pour les doigts (27) et sur sa face inférieure de pointes de contact (29, 30) et que cette plaque est reliée de façon ajustable à la partie inférieure du boîtier (12). (Fig. 1, Fig. 2, Fig. 7).

8. L'appareil d'injection à usage unique selon les revendications 1 à 7 présente la caractéristique que le boîtier (12) est relié à la plaque de fond (25) par un filetage (26), que des graduations (24a) figurent sur le boîtier de façon à ce que l'injection soit réalisée à la bonne profondeur, et que la plaque de fond (25) porte un marquage correspondant (24). (Fig. 1, Fig. 2).

9. L'appareil d'injection à usage unique selon les revendications 1 à 8 présente la caractéristique qu'un carpule (1), dont le bouchon-piston mobile et souple (1a) est dirigé vers l'extrémité supérieure de la canule, est utilisé comme réservoir. (Fig. 1 à Fig. 4).

10. L'appareil d'injection à usage unique selon la revendication 9 présente la caractéristique qu'un socle de canule saillant (6b) avec boudin d'isolation (54) est disposé sur la partie supérieure de l'élément de retenue de canule (6) enfichée dans un trou central du bouchon-piston (1a) avant l'utilisation de l'appareil ; qu'un bouchon de canule (56) hermétique mais pouvant être retiré est placé sur la partie inférieure de l'élément de retenue de canule (6), et que les éléments de verrouillage de la bague d'arrêt (19) sont montés comme un verrou double (37). (Fig. 5c, Fig. 9).

11. L'appareil d'injection à usage unique selon les revendications 1 à 8 présente la caractéristique qu'une capsule (58) avec fixation de capsule (59) est utilisée comme réservoir. (Fig. 10).

12. L'appareil d'injection à usage unique selon les revendications 1 à 11 présente la caractéristique que chaque glissoire (48, 49, 53) de l'élément de commande (3, 3a) présente une clenche (33a, 33b, 33c) - relativement au sens de rotation pour l'abaissement de la canule (7) par-delà de la position initiale (46, 47) d'une pièce coulissante (4a, 4b, 4c) de l'unité complémentaire (4). (Fig. 3, Fig. 4, Fig. 8a, Fig. 8b, Fig. 8c).

13. L'appareil d'injection à usage unique selon les revendications 1 à 12 présente la caractéristique que le réservoir (1, 58) est fermé hermétiquement au moyen d'un film de protection (31) supplémentaire sur ses côtés perçables par la canule (7). (Fig. 2, Fig. 3, Fig. 4, Fig. 10).

14. L'appareil d'injection à usage unique selon les revendications 1 à 13 présente la caractéristique qu'une cavité pour les doigts en forme de vase (10) est placée à l'extrémité du poussoir (9). (Fig. 1, Fig. 2, Fig. 11).

15. L'appareil d'injection à usage unique selon les revendications 1 à 14 présente la caractéristique que l'élément de retenue de canule (6) contient une canule simplement aiguisée supérieure (7a) et inférieure (7b), dont les chambres internes sont reliées entre elles par une jonction (32). (Fig. 2).
